# EUROPEAN PATENT APPLICATION

(11) **EP 4 851 276 A1**
(43) Date of publication of application: **09.12.2026**
(21) Application number: 25747787.7
(22) Date of filing: 24.01.2025
(51) Int. Cl.: C12N 1/20, A01G 22/40, A01N 63/22, A01N 63/20, A01N 63/27, A01P 3/00, A01P 21/00, C05F 11/08

(54) **QUALITY-IMPROVING AND NITROGEN-FIXING COUPLED MICROBIAL AGENT, AND USE THEREOF**

(30) Priority: 01.02.2024 CN 202410143849
(71) Applicant: Oil Crops Research Institute, Chinese Academy of Agricultural Sciences, Wuhan, Hubei 430062 (CN)
(72) Inventor: ZHANG, Qi, Wuhan, Hubei 430062 (CN); LI, Peiwu, Wuhan, Hubei 430062 (CN)
(74) Representative: Lorenz Seidler Gossel Part. mbB
(86) International application number: PCT/CN2025/074552
(87) International publication number: WO 2025/162219

(57) **Abstract**

Provided are a quality-improving and nitrogen-fixing coupled microbial inoculant, and use thereof. The quality-improving and nitrogen-fixing coupled microbial inoculant is a microbial composition that possesses not only a quality-improving and nitrogen-fixing coupled effect to improve quality of leguminous crop products but also effects of regulating and increasing abundance of rhizobia in rhizosphere of leguminous crops and increasing the number of nodules formed on the leguminous crops, and contains all gene sequences or 4 or more thereof among nucleotide sequences set forth in SEQ ID NOs: 1-12. When being applied to production of leguminous crops such as soybeans, peanuts, peas, broad beans, and cowpeas, the quality-improving and nitrogen-fixing coupled microbial inoculant possesses the quality-improving and nitrogen-fixing coupled effect and achieves quality improvement, nitrogen fixation, and green yield enhancement, featuring simple use, significant social and ecological benefits, and easy popularization and application.

## Description

### TECHNICAL FIELD

This disclosure relates to the field of microbiology and, in particular, to a quality-improving and nitrogen-fixing coupled microbial inoculant, and use thereof.

### BACKGROUND

Leguminous crops mainly include soybeans, peanuts, peas, broad beans, cowpeas, alfalfa, and the like, and are important sources of food and feed for humans. At present, global agriculture is facing numerous challenges in its pursuit of green and high-quality development, and the development of the leguminous crop industry is confronted with two common challenging problems: first, leguminous crops such as peanuts and soybeans are susceptible to contamination by highly toxic and strongly carcinogenic aflatoxins, which not only reduces quality and causes yield losses, but also seriously threatens human life and health. Taking aflatoxin B1 as an example, its toxicity is 10 times that of potassium cyanide, and it has been classified as a Group I carcinogen by the WHO International Agency for Research on Cancer, accounting for as high as 28.2% of global human liver cancer cases. How to improve the quality, safety, and overall quality standards of leguminous crop products has always been a hot issue in the high-quality development of the legume agriculture sector.

Second, although leguminous crops such as soybeans and peanuts have the habit of symbiotic nodulation and nitrogen fixation with soil rhizobia, under natural conditions, the number of nodules is small, the nitrogen fixation period is short (typically, there is no nodulation and nitrogen fixation during the first month of crop growth, and nodules begin to gradually decline and decay at the pod-filling or full-pod stage), and the efficiency is low. Research on biological nitrogen fixation by rhizobia has a history of more than 100 years, and has established the classic and well-recognized AON (autoregulation of nodulation) theoretical framework, i.e., plants autoregulate the number of nodules and growth, and maintain overall energy conservation. Excessive nodulation would necessarily come at the expense of plant growth. Current practices mainly involve screening and applying excellent rhizobium strains adapted to the corresponding production environments, but such applications are geographically limited and have long been constrained by the AON theory, resulting in limited improvement in nodulation and nitrogen fixation efficiency, with the best increase being about 30%, making it difficult to achieve a doubling increase in nodulation and nitrogen fixation efficiency while also significantly increasing growth (i.e., breaking the AON theoretical framework). How to improve the nodulation and nitrogen fixation efficiency of leguminous crops such as peanuts and soybeans remains a frontier challenge of international interest.

To address the aforementioned challenge, the inventors' team, after more than 20 years of continuous research, has in recent years successfully developed a quality-improving and nitrogen-fixing coupled microbial inoculant, which achieves quality improvement in leguminous crops such as soybeans and peanuts while simultaneously inducing and promoting efficient nodulation and nitrogen fixation, resulting in a doubling of yield enhancement. The quality-improving and nitrogen-fixing coupled microbial inoculant is easy to apply, low in cost, and highly effective, and features the remarkable characteristics of "two fixes" (fixing nitrogen and carbon), "three increases" (increasing yield, efficiency, and safety), and "five reductions" (reducing toxins, losses, fertilizer, costs, and carbon emissions), with great application potential. It has undergone field production demonstration trials and validation in multiple locations across the main production areas of leguminous crops such as soybeans, peanuts, and peas nationwide. This is of great significance for promoting the enhancement of soybean and oilseed production capacity and for achieving green, low-carbon, and efficient production in China.

### SUMMARY

To address the shortcomings in the prior art, this disclosure provides a quality-improving and nitrogen-fixing coupled microbial inoculant. When being applied to production of leguminous crops such as soybeans, peanuts, peas, broad beans, and cowpeas, the quality-improving and nitrogen-fixing coupled microbial inoculant possesses a quality-improving and nitrogen-fixing coupled effect and achieves quality improvement, nitrogen fixation, and green yield enhancement, featuring simple use, significant social and ecological benefits, and easy popularization and application.

To solve the above technical problems, the technical solution adopted by this disclosure is as follows:
a quality-improving and nitrogen-fixing coupled microbial inoculant, which is a microbial composition that possesses not only a quality-improving and nitrogen-fixing coupled effect to improve quality of leguminous crop products but also effects of regulating and increasing abundance of rhizobia in rhizosphere of leguminous crops and increasing the number of nodules formed on the leguminous crops, and contains all gene sequences or 4 or more thereof among nucleotide sequences set forth in SEQ ID NOs: 1-12.

According to the above solution, the quality-improving and nitrogen-fixing coupled microbial inoculant is preferably a composition of 3 kinds or more microorganisms and contains 4, 5, 6, 7, 8, 9, 10, 11, 12, or more gene sequences among the nucleotide sequences set forth in SEQ ID NOs: 1-12.

According to the above solution, sources of the microorganisms include, but are not limited to, bacteria of *Bacillus amyloliquefaciens, Bacillus subtilis, Bacillus licheniformis, Brevibacillus laterosporus, Bacillus mucilaginosus, Bacillus velezensis, Bacillus siamensis, Paenibacillus polymyxa, Paenibacillus timonensis, Pseudomonas fluorescens, Pseudomonas mendocina, Enterobacter ludwigii, Microbacterium proteolyticum, Leclercia adecarboxylata, Serratia marcescens, Empedobacter sp., Priestia megaterium,* and *Stenotrophomonas maltophilia.*

According to the above solution, the gene sequences set forth in SEQ ID NOs: 1-12 may exhibit a certain degree of variation in different strains; provided that the variation is small, for example, not more than 10% base variations, preferably not more than 5% base variations, more preferably not more than 1% base variations, i.e., sharing more than 90% identity, preferably more than 95% identity, more preferably more than 99% identity, and corresponding biological activity is retained, these sequence variants are referred to as functional equivalents of the DNA sequences set forth in SEQ ID NOs: 1-12, and containing these sequences is equivalent to containing corresponding sequences among the DNA sequences 1-12. Provided that the microbial composition contains all of the gene sequences or 4 or more thereof among the nucleotide sequences set forth in SEQ ID NOs: 1-12, or functional equivalents of these gene sequences, and possesses not only the quality-improving and nitrogen-fixing coupled effect of the microbial inoculant to improve quality of leguminous crop products but also the effects of regulating and increasing abundance of rhizobia in rhizosphere of leguminous crops and increasing the number of nodules formed on the leguminous crops, the microbial composition constitutes the quality-improving and nitrogen-fixing coupled microbial inoculant of this disclosure.

According to the above solution, the quality-improving and nitrogen-fixing coupled microbial inoculant is capable of improving the quality of leguminous crop products by increasing content of one or more nutritional and functional components, such as protein, glutamic acid, peanut resveratrol, and soybean isoflavone. According to the above solution, the quality-improving and nitrogen-fixing coupled microbial inoculant possesses an alleviating effect, namely a control effect, on one or more soil-borne diseases such as pod rot in peanuts, stay-green syndrome in soybeans, and southern blight, bacterial wilt, root rot, and *Sclerotinia* disease in leguminous crops.

According to the above solution, the quality-improving and nitrogen-fixing coupled microbial inoculant is capable of reducing an aflatoxin level of crop products, thereby improving quality and safety of leguminous crop products.

According to the above solution, the quality-improving and nitrogen-fixing coupled microbial inoculant is capable of reducing spots on peanut pod surfaces.

The quality-improving and nitrogen-fixing coupled microbial inoculant is capable of promoting early nodulation of leguminous crops such as peanuts and soybeans, and prolonging a nodulation and nitrogen fixation period.

The above DNA sequences 1-12 are specific sequences obtained after comparison with the genomic big data from GenBank, and have a direct or indirect correlation with the functional effects of the quality-improving and nitrogen-fixing coupled microbial inoculant of this disclosure. Provided that the microbial composition contains more than 4 of the above DNA sequences 1-12 and possesses the quality-improving and nitrogen-fixing coupled effect to improve quality of leguminous crop products, the microbial composition constitutes the quality-improving and nitrogen-fixing coupled microbial inoculant of this disclosure; further, it can also promote early nodulation of leguminous crops, prolong the nodulation and nitrogen fixation period, and increase the yield per unit area and total biomass of leguminous crops. These genes may exhibit a certain degree of variation in different strains; provided that the degree of variation is small, for example, not more than 10% base variations, and they retain corresponding biological activity, containing them is equivalent to containing the corresponding DNA sequences set forth in SEQ ID NOs: 1-12, and they are referred to as functional equivalents of the DNA sequences set forth in SEQ ID NOs: 1-12; and containing them is also equivalent to containing all of the gene sequences or 4 or more thereof among the DNA sequences 1-12 set forth in SEQ ID NOs: 1-12.

The quality-improving and nitrogen-fixing coupled microbial inoculant provided by this disclosure may be, but is not limited to, a composition of three or more of the following microbial strains: *Bacillus subtilis* with an accession number of CCTCC NO: M 20231597, *Bacillus velezensis* with an accession number of CCTCC NO: M 20231600, *Brevibacillus laterosporus* with an accession number of CCTCC NO: M 20231808, *Bacillus amyloliquefaciens* with an accession number of CCTCC NO: M 20231810, *Enterobacter ludwigii* with an accession number of CCTCC NO: M 20231812, and *Microbacterium proteolyticum* with an accession number of CCTCC NO: M 20231814. Specifically, it may be a combination of three, four, five, or six of the above microbial strains.

The quality-improving and nitrogen-fixing coupled microbial inoculant provided by this disclosure may also be a combination of three or more of the above microbial strains with other microorganisms, such that a combined microbial inoculant contains all of the gene sequences or 4 or more thereof among the nucleotide sequences set forth in SEQ ID NOs: 1-12, or functional equivalents thereof, and possesses not only the quality-improving and nitrogen-fixing coupled effect of the microbial inoculant as described above but also the effects of regulating and increasing abundance of rhizobia in rhizosphere of leguminous crops and increasing the number of nodules formed on the leguminous crops, constituting the quality-improving and nitrogen-fixing coupled microbial inoculant of this disclosure.

The quality-improving and nitrogen-fixing coupled microbial inoculant provided by this disclosure may also be a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with one or more of the following three strains: *Bacillus subtilis* with an accession number of CCTCC NO: M 20231597, *Bacillus velezensis* with an accession number of CCTCC NO: M 20231600, and *Microbacterium proteolyticum* with an accession number of CCTCC NO: M 20231814, for example, a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with *Bacillus subtilis* with an accession number of CCTCC NO: M 20231597, or a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with *Bacillus velezensis* with an accession number of CCTCC NO: M 20231600, or a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with *Microbacterium proteolyticum* with an accession number of CCTCC NO: M 20231814, such that a combined microbial inoculant contains all of the gene sequences or 4 or more thereof among the nucleotide sequences set forth in SEQ ID NOs: 1-12, or functional equivalents thereof, and possesses not only the quality-improving and nitrogen-fixing coupled effect of the microbial inoculant as described above to improve quality of leguminous crop products but also the effects of regulating and increasing abundance of rhizobia in rhizosphere of leguminous crops and increasing the number of nodules formed on the leguminous crops, constituting the quality-improving and nitrogen-fixing coupled microbial inoculant of this disclosure.

The quality-improving and nitrogen-fixing coupled microbial inoculant provided by this disclosure may also be a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with other microorganisms, such that a combined microbial inoculant contains all of the gene sequences or 4 or more thereof among the nucleotide sequences set forth in SEQ ID NOs: 1-12, or functional equivalents thereof, and possesses not only the quality-improving and nitrogen-fixing coupled effect of the microbial inoculant as described above to improve quality of leguminous crop products but also the effects of regulating and increasing abundance of rhizobia in rhizosphere of leguminous crops and increasing the number of nodules formed on the leguminous crops, constituting the quality-improving and nitrogen-fixing coupled microbial inoculant of this disclosure.

The *Bacillus subtilis* was deposited at the China Center for Type Culture Collection, Wuhan University located at Wuhan, China on September 04, 2023 with an accession number of CCTCC NO: M 20231597, and is taxonomically named as *Bacillus subtilis* AR1003.

The *Bacillus velezensis* was deposited at the China Center for Type Culture Collection, Wuhan University located at Wuhan, China on September 04, 2023 with an accession number of CCTCC NO: M 20231600, and is taxonomically named as *Bacillus velezensis* AR1006.

The *Brevibacillus laterosporus* was deposited at the China Center for Type Culture Collection, Wuhan University located at Wuhan, China on September 27, 2023 with an accession number of CCTCC NO: M 20231808, and is taxonomically named *as Brevibacillus laterosporus strain D-CB0402*.

The *Bacillus amyloliquefaciens* was deposited at the China Center for Type Culture Collection, Wuhan University located at Wuhan, China on September 27, 2023 with an accession number of CCTCC NO: M 20231810, and is taxonomically named as *Bacillus amyloliquefaciens strain* D-JDFN02.

The *Enterobacter ludwigii* was deposited at the China Center for Type Culture Collection, Wuhan University located at Wuhan, China on September 27, 2023 with an accession number of CCTCC NO: M 20231812, and is taxonomically named *Enterobacter ludwigii strain* D-LD1601.

The *Microbacterium proteolyticum* was deposited at the China Center for Type Culture Collection, Wuhan University located at Wuhan, China on September 27, 2023 with an accession number of CCTCC NO: M 20231814, and is taxonomically named *Microbacterium proteolyticum strain D-WG1701*.

According to the above solution, a percentage of viable cells of any single strain in the microbial inoculant as described above, specifically a microbial composition formed by mixing, is greater than or equal to 1%.

The quality-improving and nitrogen-fixing coupled microbial inoculant provided by this disclosure is a microbial composition that produces a quality-improving and nitrogen-fixing coupled effect through a synergistic action of constituent microorganisms in the microbial composition. When used in crop production, it possesses not only the quality-improving and nitrogen-fixing coupled effect but also effects of regulating and increasing abundance of rhizobia in rhizosphere of leguminous crops and increasing the number of nodules formed on the leguminous crops. Although it is not a rhizobium, it can exert the quality-improving and nitrogen-fixing coupled effect to improve quality of leguminous crop products, regulate and increase abundance of rhizobia in rhizosphere of leguminous crops, increase the number of nodules formed on the leguminous crops, enhance nitrogenase activity per plant, promote early nodulation of leguminous crops such as peanuts and soybeans, prolong the nodulation and nitrogen fixation period, and increase the yield per unit area and total biomass of leguminous crops.

The quality-improving and nitrogen-fixing coupled microbial inoculant as described above can be prepared by combining microorganisms in the microbial inoculant and subjecting them to fermentation. The fermentation can employ conventional fermentation methods for bacteria or fungi that are known in the prior art, including those disclosed in existing literature.

In a second aspect, this disclosure provides use of the quality-improving and nitrogen-fixing coupled microbial inoculant as described above in production of leguminous crops for the following purposes: improving quality and safety of leguminous crop products; promoting nodulation and nitrogen fixation of the leguminous crops; increasing a yield per unit area of the leguminous crops; recruiting indigenous rhizobia and increasing abundance of rhizobia in rhizosphere soil of the leguminous crops; promoting earlier nodulation and prolonging a nodulation and nitrogen fixation period of the leguminous crops; preventing nutrient deficiency and premature senescence of the leguminous crops at maturity; increasing the number of pods of the leguminous crops; increasing fullness of pods and reducing a rate of shriveled pods of the leguminous crops; promoting earlier flowering and earlier podding of the leguminous crops; reducing occurrence of pod rot in peanuts; reducing occurrences of bacterial wilt in the leguminous crops; reducing occurrences of powdery mildew in the leguminous crops; reducing occurrences of leaf spot in the leguminous crops; reducing occurrences of root-knot nematode disease in the leguminous crops; reducing occurrences of root rot in the leguminous crops; reducing occurrences of *Sclerotinia* disease in the leguminous crops; reducing occurrences of downy mildew in the leguminous crops; reducing occurrences of *Fusarium* wilt in the leguminous crops, and reducing occurrences of southern blight in the leguminous crops; reducing severity of stay-green syndrome in soybeans; promoting carbon emission reduction in the leguminous crops, and facilitating soil improvement; promoting an increase in total biomass of the leguminous crops; reducing spots on peanut pod surfaces and improving marketability; and promoting yield enhancement of soybeans in saline-alkali land.

In a third aspect, this disclosure provides use of the quality-improving and nitrogen-fixing coupled microbial inoculant as described above for preparing a quality-improving and nitrogen-fixing coupled microbial compound fertilizer, or a quality-improving and nitrogen-fixing coupled microbial organic fertilizer, or a quality-improving and nitrogen-fixing coupled microbial micronutrient fertilizer, or a quality-improving and nitrogen-fixing coupled microbial moisture-retaining fertilizer, or a quality-improving and nitrogen-fixing coupled microbial inorganic-organic compound fertilizer, or a quality-improving and nitrogen-fixing coupled microbial seed coating agent/seed dressing agent/seed soaking agent to simultaneously exert effects of the quality-improving and nitrogen-fixing coupled microbial inoculant in production.

This disclosure further provides a quality-improving and nitrogen-fixing coupled microbial compound fertilizer, or a quality-improving and nitrogen-fixing coupled microbial organic fertilizer, or a quality-improving and nitrogen-fixing coupled microbial micronutrient fertilizer, or a quality-improving and nitrogen-fixing coupled microbial moisture-retaining fertilizer, or a quality-improving and nitrogen-fixing coupled microbial inorganic-organic compound fertilizer, or a quality-improving and nitrogen-fixing coupled microbial rhizobium fertilizer, or a quality-improving and nitrogen-fixing coupled microbial seed coating agent/seed dressing agent/seed soaking agent prepared by using the quality-improving and nitrogen-fixing coupled microbial inoculant as described above.

In a fourth aspect, this disclosure provides a method for preparing a quality-improving and nitrogen-fixing coupled microbial compound fertilizer, or a quality-improving and nitrogen-fixing coupled microbial organic fertilizer, or a quality-improving and nitrogen-fixing coupled microbial micronutrient fertilizer, or a quality-improving and nitrogen-fixing coupled microbial moisture-retaining fertilizer, or a quality-improving and nitrogen-fixing coupled microbial inorganic-organic compound fertilizer, or a quality-improving and nitrogen-fixing coupled microbial rhizobium fertilizer, or a quality-improving and nitrogen-fixing coupled microbial seed coating agent/seed dressing agent/seed soaking agent, by using the quality-improving and nitrogen-fixing coupled microbial inoculant as described above, where
the method for preparing the quality-improving and nitrogen-fixing coupled microbial compound fertilizer includes: blending a compound fertilizer with the quality-improving and nitrogen-fixing coupled microbial inoculant according to a conventional amount of the compound fertilizer and an amount of the quality-improving and nitrogen-fixing coupled microbial inoculant of not less than 80 billion viable cells per mu of land, and then adsorbing and immobilizing quality-improving and nitrogen-fixing coupled microbial inoculant onto compound fertilizer particles by a physical method to prepare the quality-improving and nitrogen-fixing coupled microbial compound fertilizer;
the method for preparing the quality-improving and nitrogen-fixing coupled microbial organic fertilizer includes: blending an organic fertilizer with the quality-improving and nitrogen-fixing coupled microbial inoculant according to a conventional amount of the organic fertilizer and an amount of the quality-improving and nitrogen-fixing coupled microbial inoculant of not less than 80 billion viable cells per mu of land, and then adsorbing and immobilizing quality-improving and nitrogen-fixing coupled microbial inoculant onto organic fertilizer particles by a physical method to prepare the quality-improving and nitrogen-fixing coupled microbial organic fertilizer;
the method for preparing the quality-improving and nitrogen-fixing coupled microbial micronutrient fertilizer includes: blending a micronutrient fertilizer with the quality-improving and nitrogen-fixing coupled microbial inoculant according to a conventional amount of the micronutrient fertilizer and an amount of the quality-improving and nitrogen-fixing coupled microbial inoculant of not less than 80 billion viable cells per mu of land, uniformly mixing them by a physical method, and adsorbing and immobilizing quality-improving and nitrogen-fixing coupled microbial inoculant onto micronutrient fertilizer particles to prepare the quality-improving and nitrogen-fixing coupled microbial micronutrient fertilizer;
the method for preparing the quality-improving and nitrogen-fixing coupled microbial moisture-retaining fertilizer includes: blending a water retaining agent with the quality-improving and nitrogen-fixing coupled microbial inoculant according to a conventional amount of the water retaining agent and an amount of the quality-improving and nitrogen-fixing coupled microbial inoculant of not less than 80 billion viable cells per mu of land, and then uniformly mixing them by a conventional physical method, or adsorbing and immobilizing quality-improving and nitrogen-fixing coupled microbial inoculant onto the water retaining agent to prepare the quality-improving and nitrogen-fixing coupled microbial moisture-retaining fertilizer;
the method for preparing the quality-improving and nitrogen-fixing coupled microbial rhizobium fertilizer includes: blending a rhizobium fertilizer with the quality-improving and nitrogen-fixing coupled microbial inoculant according to a conventional amount of the rhizobium fertilizer and an amount of the quality-improving and nitrogen-fixing coupled microbial inoculant of not less than 80 billion viable cells per mu of land, then uniformly mixing them by a conventional physical method, or uniformly mixing them and then immobilizing them onto carrier particles to prepare the quality-improving and nitrogen-fixing coupled microbial rhizobium fertilizer;
the method for preparing the quality-improving and nitrogen-fixing coupled microbial inorganic-organic compound fertilizer includes: blending an inorganic-organic compound fertilizer with the quality-improving and nitrogen-fixing coupled microbial inoculant according to a conventional amount of the inorganic-organic compound fertilizer and an amount of the quality-improving and nitrogen-fixing coupled microbial inoculant of not less than 80 billion viable cells per mu of land, and then adsorbing and immobilizing quality-improving and nitrogen-fixing coupled microbial inoculant onto inorganic-organic particles by a physical method to prepare the quality-improving and nitrogen-fixing coupled microbial inorganic-organic compound fertilizer; and
the method for preparing the quality-improving and nitrogen-fixing coupled microbial seed coating agent/seed dressing agent/seed soaking agent includes: blending a seed coating agent or seed dressing agent or seed soaking agent with the quality-improving and nitrogen-fixing coupled microbial inoculant according to an amount of the seed coating agent or seed dressing agent or seed soaking agent and an amount of the quality-improving and nitrogen-fixing coupled microbial inoculant of not less than 80 billion viable cells per mu of land, and then uniformly mixing them by a conventional physical method to prepare the quality-improving and nitrogen-fixing coupled microbial seed coating agent/seed dressing agent/seed soaking agent.

In a fifth aspect, this disclosure provides use of the quality-improving and nitrogen-fixing coupled microbial compound fertilizer, or the quality-improving and nitrogen-fixing coupled microbial organic fertilizer, or the quality-improving and nitrogen-fixing coupled microbial micronutrient fertilizer, or the quality-improving and nitrogen-fixing coupled microbial moisture-retaining fertilizer, or the quality-improving and nitrogen-fixing coupled microbial inorganic-organic compound fertilizer, or the quality-improving and nitrogen-fixing coupled microbial rhizobium fertilizer, or the quality-improving and nitrogen-fixing coupled microbial seed coating agent/seed dressing agent/seed soaking agent prepared by using the quality-improving and nitrogen-fixing coupled microbial inoculant as described above in crop production for any one of the following purposes: improving quality and safety of leguminous crop products; promoting nodulation and nitrogen fixation of leguminous crops; increasing a yield per unit area of the leguminous crops; recruiting indigenous rhizobia and increasing abundance of rhizobia in rhizosphere soil of the leguminous crops; promoting earlier nodulation and prolonging a nodulation and nitrogen fixation period of the leguminous crops; preventing nutrient deficiency and premature senescence of the leguminous crops at maturity; increasing the number of pods of the leguminous crops; increasing fullness of pods and reducing a rate of shriveled pods of the leguminous crops; promoting earlier flowering and earlier podding of the leguminous crops; reducing occurrence of pod rot in peanuts; reducing occurrences of bacterial wilt in the leguminous crops; reducing occurrences of powdery mildew in the leguminous crops; reducing occurrences of leaf spot in the leguminous crops; reducing occurrences of root-knot nematode disease in the leguminous crops; reducing occurrences of root rot in the leguminous crops; reducing occurrences of *Sclerotinia* disease in the leguminous crops; reducing occurrences of downy mildew in the leguminous crops; reducing occurrences of *Fusarium* wilt in the leguminous crops, and reducing occurrences of southern blight in the leguminous crops; reducing severity of stay-green syndrome in soybeans; promoting carbon emission reduction in leguminous crops, and facilitating soil improvement; promoting an increase in total biomass of the leguminous crops; reducing spots on peanut pod surfaces and improving marketability; and promoting yield enhancement of soybeans in saline-alkali land.

In a sixth aspect, this disclosure provides a method for production of leguminous crops, including: selecting and preparing a microbial inoculant, such that the microbial inoculant, upon analytical determination, contains all gene sequences or 4 or more thereof among nucleotide sequences set forth in SEQ ID NOs: 1-12, and possesses not only a quality-improving and nitrogen-fixing coupled effect to improve quality of leguminous crop products but also effects of regulating and increasing abundance of rhizobia in rhizosphere of the leguminous crops and increasing the number of nodules formed on the leguminous crops; and applying the microbial inoculant to crops for quality improvement, nitrogen fixation, and green yield enhancement.

According to the above solution, the gene sequences set forth in SEQ ID NOs: 1-12 may exhibit not more than 10% base variations in different strains; provided that the sequences share more than 90% identity with corresponding DNA sequences and retain corresponding biological activity, these sequence variants are considered equivalent to containing corresponding gene sequences among the DNA sequences 1-12 set forth in SEQ ID NOs: 1-12, and are referred to as functional equivalents of the DNA sequences set forth in SEQ ID NOs: 1-12. Provided that the microbial inoculant contains all of the gene sequences or 4 or more thereof among the nucleotide sequences set forth in SEQ ID NOs: 1-12, or functional equivalents of these gene sequences, and possesses not only the quality-improving and nitrogen-fixing coupled effect of the microbial inoculant as described above to improve quality of leguminous crop products but also the effects of regulating and increasing abundance of rhizobia in rhizosphere of the leguminous crops and increasing the number of nodules formed on the leguminous crops, the microbial inoculant constitutes the quality-improving and nitrogen-fixing coupled microbial inoculant of this disclosure, and can be applied in crops for quality improvement, nitrogen fixation, and green yield enhancement.

According to the above solution, the gene sequences set forth in SEQ ID NOs: 1-12 may exhibit a certain degree of variation in different strains; provided that the variation is small, for example, not more than 10% base variations, preferably not more than 5% base variations, more preferably not more than 1% base variations, i.e., sharing more than 90% identity, preferably more than 95% identity, more preferably more than 99% identity, and corresponding biological activity is retained, these sequence variants are referred to as functional equivalents of the DNA sequences set forth in SEQ ID NOs: 1-12, and containing these sequences is equivalent to containing corresponding sequences among the DNA sequences 1-12. Provided that the microbial composition contains all of the gene sequences or 4 or more thereof among the nucleotide sequences set forth in SEQ ID NOs: 1-12, or functional equivalents of these gene sequences, and possesses not only the quality-improving and nitrogen-fixing coupled effect of the microbial inoculant to improve quality of leguminous crop products but also the effects of regulating and increasing abundance of rhizobia in rhizosphere of leguminous crops and increasing the number of nodules formed on the leguminous crops, the microbial composition constitutes the quality-improving and nitrogen-fixing coupled microbial inoculant of this disclosure.

In the above solution, the quality-improving and nitrogen-fixing coupled microbial inoculant is capable of improving the quality of leguminous crop products by increasing content of one or more nutritional and functional components, such as protein, glutamic acid, peanut resveratrol, and soybean isoflavone. According to the above solution, the quality-improving and nitrogen-fixing coupled microbial inoculant possesses an alleviating effect, namely a control effect, on one or more soil-borne diseases such as pod rot in peanuts, stay-green syndrome in soybeans, and southern blight, bacterial wilt, root rot, and *Sclerotinia* disease in leguminous crops.

In the above solution, the quality-improving and nitrogen-fixing coupled microbial inoculant is capable of reducing an aflatoxin level of crop products, thereby improving quality and safety of leguminous crop products.

In the above solution, the quality-improving and nitrogen-fixing coupled microbial inoculant is capable of reducing spots on peanut pod surfaces.

According to the above solution, the quality-improving and nitrogen-fixing coupled microbial inoculant is preferably a composition of 3 kinds or more microorganisms and contains 4, 5, 6, 7, 8, 9, 10, 11, 12, or more gene sequences among the nucleotide sequences set forth in SEQ ID NOs: 1-12.

In the above solution, the quality-improving and nitrogen-fixing coupled microbial inoculant may be, but is not limited to, a composition of three or more of the following microbial strains: *Bacillus subtilis* with an accession number of CCTCC NO: M 20231597, *Bacillus velezensis* with an accession number of CCTCC NO: M 20231600, *Brevibacillus laterosporus* with an accession number of CCTCC NO: M 20231808, *Bacillus amyloliquefaciens* with an accession number of CCTCC NO: M 20231810, *Enterobacter ludwigii* with an accession number of CCTCC NO: M 20231812, and *Microbacterium proteolyticum* with an accession number of CCTCC NO: M 20231814. Specifically, it may be a combination of three, four, five, or six of the above microbial strains.

In the above solution, the quality-improving and nitrogen-fixing coupled microbial inoculant may also be a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with one or more of the following three strains: *Bacillus subtilis* with an accession number of CCTCC NO: M 20231597, *Bacillus velezensis* with an accession number of CCTCC NO: M 20231600, and *Microbacterium proteolyticum* with an accession number of CCTCC NO: M 20231814, for example, a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with *Bacillus subtilis* with an accession number of CCTCC NO: M 20231597, or a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with *Bacillus velezensis* with an accession number of CCTCC NO: M 20231600, or a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with *Microbacterium proteolyticum* with an accession number of CCTCC NO: M 20231814, such that a combined microbial inoculant contains all of the gene sequences or 4 or more thereof among the nucleotide sequences set forth in SEQ ID NOs: 1-12, or functional equivalents thereof, and possesses not only the quality-improving and nitrogen-fixing coupled effect of the microbial inoculant as described above to improve quality of leguminous crop products but also the effects of regulating and increasing abundance of rhizobia in rhizosphere of leguminous crops and increasing the number of nodules formed on the leguminous crops, constituting the quality-improving and nitrogen-fixing coupled microbial inoculant of this disclosure.

In the above solution, the quality-improving and nitrogen-fixing coupled microbial inoculant may also be a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with other microorganisms, such that a combined microbial inoculant contains all of the gene sequences or 4 or more thereof among the nucleotide sequences set forth in SEQ ID NOs: 1-12, or functional equivalents thereof, and possesses not only the quality-improving and nitrogen-fixing coupled effect of the microbial inoculant as described above to improve quality of leguminous crop products but also the effects of regulating and increasing abundance of rhizobia in rhizosphere of leguminous crops and increasing the number of nodules formed on the leguminous crops, constituting the quality-improving and nitrogen-fixing coupled microbial inoculant of this disclosure.

In the above solution, a percentage of viable cells of any single strain in the microbial inoculant as described above, specifically a microbial composition formed by mixing, is greater than or equal to 1%.

The beneficial effects of this disclosure are as follows:
1.the quality-improving and nitrogen-fixing coupled microbial inoculant can be used to increase the production of leguminous crops such as peanuts and soybeans. Through a single technology, it improves the quality of leguminous crop products, and further alleviates diseases in leguminous crops and/or enhances quality and safety of crop products and/or reduces spots on peanut pod surfaces, while simultaneously inducing and promoting efficient nodulation and nitrogen fixation in soybeans and peanuts, as well as extremely significant yield enhancement; 2. its application is simple, low in cost, and highly effective; 3. it is of great significance for boosting the production capacity of soybeans and oilseeds, as well as for achieving high-quality development and green, low-carbon, and efficient production of soybeans and oilseeds in China.

### DETAILED DESCRIPTION OF THE EMBODIMENTS

### Example 1: Isolation and Identification of Microbial Strains (a Series of Microbial Strains for Combined Use)

A series of microbial strains were obtained through the following isolation and identification steps.
(1) Intact plants and rhizosphere soil samples of peanuts, soybeans, peas, broad beans, cowpeas, and alfalfa were taken, ground, and mixed thoroughly. Strains were isolated by conventional bacterial isolation methods, and then identified by conventional 16S rDNA methods. Through the above operations, a series of bacteria were obtained, including *Bacillus amyloliquefaciens*, *Bacillus subtilis*, *Bacillus licheniformis*, *Brevibacillus laterosporus*, *Bacillus mucilaginosus*, *Bacillus velezensis*, *Bacillus siamensis*, *Paenibacillus polymyxa*, *Paenibacillus timonensis*, *Pseudomonas fluorescens*, *Pseudomonas mendocina*, *Enterobacter ludwigii*, *Microbacterium proteolyticum*, *Leclercia adecarboxylata*, *Serratia marcescens*, *Empedobacter sp.*, *Priestia megaterium*, and *Stenotrophomonas maltophilia*. For detailed information, see Table 1.

**Table 1. List of Strains Isolated and Identified from Mixtures of Major Leguminous Crops Such as Peanuts and Soybeans**

| Strain code or accession number | Strain name | Strain code or accession number | Strain name |
|---|---|---|---|
| CCTCC NO: M 20231597 | *Bacillus subtilis* | CCTCC NO: M 20231600 | *Bacillus velezensis* |
| CCTCC NO: M 20231808 | *Brevibacillus laterosporus* | CCTCC NO: M 20231810 | *Bacillus amyloliquefaciens* |
| CCTCC NO: M 20231812 | *Enterobacter ludwigii* | CCTCC NO: M 20231814 | *Microbacterium proteolyticum* |
| Strain 207 | *Paenibacillus timonensis* | Strain 208 | *Enterobacter ludwigii* |
| Strain 209 | *Stenotrophomonas maltophilia* | Strain 210 | *Bacillus mucilaginosus* |
| Strain 211 | *Bacillus siamensis* | Strain 212 | *Bacillus amyloliquefaciens* |
| Strain 213 | *Bacillus subtilis* | Strain 214 | *Bacillus licheniformis* |
| Strain 215 | *Brevibacillus laterosporus* | Strain 216 | *Bacillus mucilaginosus* |
| Strain 217 | *Paenibacillus polymyxa* | Strain 218 | *Paenibacillus timonensis* |
| Strain 219 | *Pseudomonas fluorescens* | Strain 220 | *Pseudomonas mendocina* |
| Strain 221 | *Leclercia adecarboxylata* | Strain 222 | *Serratia marcescens* |
| Strain 223 | *Empedobacter sp.* | Strain 224 | *Priestia megaterium* |
| Strain 225 | *Stenotrophomonas maltophilia* | Strain 226 | *Enterobacter ludwigii* |
| Strain 227 | *Bacillus velezensis* | Strain 228 | *Brevibacillus laterosporus* |
| Strain 229 | *Bacillus amyloliquefaciens* | Strain 230 | *Microbacterium proteolyticum* |
| Strain 231 | Not *Rhizobium*; genus and species unidentified | Strain 232 | Not *Rhizobium*; genus and species unidentified |
| Strain 233 | Not *Rhizobium*; genus and species unidentified | Strain 234 | Not *Rhizobium*; genus and species unidentified |
| Strain 235 | Not *Rhizobium*; genus and species unidentified | Strain 236 | Not *Rhizobium*; genus and species unidentified |
| Strain 237 | Not *Rhizobium*; genus and species unidentified | Strain 238 | Not *Rhizobium*; genus and species unidentified |
| Strain 239 | Not *Rhizobium*; genus and species unidentified | Strain 240 | Not *Rhizobium*; genus and species unidentified |
| Strain 241 | Not *Rhizobium*; genus and species unidentified | Strain 242 | Not *Rhizobium*; genus and species unidentified |

### Example 2: Preparation of Microorganisms and Compositions Thereof

The series of bacterial strains obtained above were scaled up and cultured by conventional bacterial media to prepare bulk fermentation broths or bacterial powders of the above bacterial strains.

The fermentation broths or bacterial powders of the above bacterial strains were mixed individually (i.e., one strain) or in combinations of two or more strains to form a series of microbial compositions. The information of these microbial compositions is shown in Table 2. In each microbial composition, the percentage of viable cells of any single strain was greater than or equal to 1%.

**Table 2. Information of Microorganisms or Microbial Compositions**

| Composition No. | Constituent strains and ratio (%) | Composition No. | Constituent strains and ratio |
|---|---|---|---|
| Microbial inoculant 1 | Constituents: CCTCC NO: M 20231597/CCTCC NO: M 20231808/CCTCC NO: M 20231812/CCTCC NO: M 20231810 | Microbial inoculant 2 | Constituents: CCTCC NO: M 20231597/CCTCC NO: M 20231808/CCTCC NO: M 20231812/CCTCC NO: M 20231810 |
| | Ratio: 30/30/30/10 | | Ratio: 30/30/10/30 |
| Microbial inoculant 3 | Constituents: CCTCC NO: M 20231597/CCTCC NO: M 20231808/CCTCC NO: M 20231812/CCTCC NO: M 20231810 | Microbial inoculant 4 | Constituents: CCTCC NO: M 20231597/CCTCC NO: M 20231808/CCTCC NO: M 20231812/CCTCC NO: M 20231810 |
| | Ratio: 30/10/30/30 | | Ratio: 10/30/30/30 |
| Microbial inoculant 5 | Constituents: CCTCC NO: M 20231597/CCTCC NO: M 20231808/CCTCC NO: M 20231812/CCTCC NO: M 20231810 | Microbial inoculant 6 | Constituents: CCTCC NO: M 20231597/CCTCC NO: M 20231808/CCTCC NO: M 20231812/CCTCC NO: M 20231810 |
| | Ratio: 33/33/33/1 | | Ratio: 33/33/1/33 |
| Microbial inoculant 7 | Constituents: CCTCC NO: M 20231597/CCTCC NO: M 20231808/CCTCC NO: M 20231812/CCTCC NO: M 20231810 | Microbial inoculant 8 | Constituents: CCTCC NO: M 20231597/CCTCC NO: M 20231808/CCTCC NO: M 20231812/CCTCC NO: M 20231810 |
| | Ratio: 33/1/33/33 | | Ratio: 1/33/33/33 |
| Microbial inoculant 9 | Constituents: CCTCC NO: M 20231814/CCTCC NO: M 20231808/CCTCC NO: M 20231812/CCTCC NO: M 20231810 | Microbial inoculant 10 | Constituents: CCTCC NO: M 20231600/CCTCC NO: M 20231808/CCTCC NO: M 20231812/CCTCC NO: M 20231810 |
| | Ratio: 30/30/20/20 | | Ratio: 30/30/30/10 |
| Microbial inoculant 11 | Constituents: CCTCC NO: M 20231808/CCTCC NO: M 20231812/CCTCC NO: M 20231810/Strain 207 | Microbial inoculant 12 | Constituents: Strain 209/CCTCC NO: M 20231808/CCTCC NO: M 20231812/CCTCC NO: M 20231810 |
| | Ratio: 30/30/30/10 | | Ratio: 30/30/30/10 |
| Microbial inoculant 13 | Constituents: Strain 210/CCTCC NO: M 20231808/CCTCC NO: M 20231812/CCTCC NO: M 20231810 | Microbial inoculant 14 | Constituents: Strain 211/CCTCC NO: M 20231808/CCTCC NO: M 20231812/CCTCC NO: M 20231810 |
| | Ratio: 30/1/30/10/29 | | Ratio: 30/30/30/10 |
| Microbial inoculant 15 | Constituents: Strain 213/CCTCC NO: M 20231812/CCTCC NO: M 20231810 | Microbial inoculant 16 | Constituents: Strain 214/CCTCC NO: M 20231808/CCTCC NO: M 20231812/CCTCC NO: M 20231810 |
| | Ratio: 40/40/20 | | Ratio: 30/30/30/10 |
| Microbial inoculant 17 | Constituents: Strain 217/CCTCC NO: M 20231808/CCTCC NO: M 20231812/CCTCC NO: M 20231810 | Microbial inoculant 18 | Constituents: Strain 218/CCTCC NO: M 20231808/CCTCC NO: M 20231812/CCTCC NO: M 20231810 |
| | Ratio: 30/30/30/10 | | Ratio: 30/30/30/10 |
| Microbial inoculant 19 | Constituents: Strain 219/CCTCC NO: M 20231808/CCTCC NO: M 20231812/CCTCC NO: M 20231810 | Microbial inoculant 20 | Constituents: CCTCC NO: M 20231808/CCTCC NO: M 20231812/CCTCC NO: M 20231810/Strain 220 |
| | Ratio: 30/30/30/10 | | Ratio: 30/30/30/10 |
| Microbial inoculant 21 | Constituents: Strain 221/CCTCC NO: M 20231808/CCTCC NO: M 20231812/CCTCC NO: M 20231810 | Microbial inoculant 22 | Constituents: Strain 222/CCTCC NO: M 20231808/CCTCC NO: M 20231812/CCTCC NO: M 20231810 |
| | Ratio: 30/30/30/10 | | Ratio: 30/30/30/10 |
| Microbial inoculant 23 | Constituents: Strain 223/CCTCC NO: M 20231808/CCTCC NO: M 20231812/CCTCC NO: M 20231810 | Microbial inoculant 24 | Constituents: Strain 224/CCTCC NO: M 20231808/CCTCC NO: M 20231812/CCTCC NO: M 20231810 |
| | Ratio: 30/30/30/10 | | Ratio: 30/30/30/10 |
| Microbial inoculant 25 | Constituents: Strain 225/CCTCC NO: M 20231808/CCTCC NO: M 20231812/CCTCC NO: M 20231810 | Microbial inoculant 26 | Constituents: Strain 208/Strain 210/Strain 212/Strain 214 |
| | Ratio: 30/30/30/10 | | Ratio: 30/30/30/10 |
| Microbial inoculant 27 | Constituents: Strain 215/Strain 216/Strain 226 | Microbial inoculant 28 | Constituents: Strain 226/Strain 227/Strain 228/Strain 229 |
| | Ratio: 30/30/30/10 | | Ratio: 30/30/30/10 |
| Microbial inoculant 29 | Constituents: Strain 230/Strain 231/Strain 232 | Microbial inoculant 30 | Constituents: Strain 233/Strain 234 |
| | Ratio: 45/45/10 | | Ratio: 90/10 |
| Microbial inoculant 31 | Constituents: Strain 235/Strain 236 | Microbial inoculant 32 | Constituents: Strain 217 |
| | Ratio: 50/50 | | Ratio: 100 |
| Microbial inoculant 33 | Constituents: Strain 218 | Microbial inoculant 34 | Constituents: Strain 219 |
| | Ratio: 100 | | Ratio: 100 |
| Microbial inoculant 35 | Constituents: Strain 220 | Microbial inoculant 36 | Constituents: Strain 221 |
| | Ratio: 100 | | Ratio: 100 |
| Microbial inoculant 37 | Constituents: Strain 222 | Microbial inoculant 38 | Constituents: Strain 223 |
| | Ratio: 100 | | Ratio: 100 |
| Microbial inoculant 39 | Constituents: Strain 224 | Microbial inoculant 40 | Constituents: Strain 225 |
| | Ratio: 100 | | Ratio: 100 |
| Microbial inoculant 41 | Constituents: Strain 236/Strain 239/Strain 240 | Microbial inoculant 42 | Constituents: Strain 237/Strain 238/Strain 241/Strain 242 |
| | Ratio: 30/30/40 | | Ratio: 25/25/25/25 |

### Example 3: Sequencing of the Microbial Inoculant

A sufficient number of samples were taken from the microorganisms or microbial compositions in Table 2 of Example 2. Total DNA was sequentially extracted from these samples by conventional DNA extraction methods, and the DNA sequences of these samples were determined by conventional DNA sequencing methods. Finally, the homology between the above-determined DNA sequences and the gene sequences provided herein was compared by conventional analytical methods.

The results of the above homology analysis are shown in Table 3.

**Table 3. Information of Microorganisms or Microbial Compositions**

| Microorganism or composition No. | Number of characteristic genes among the 12 gene sequences provided herein (only those with over 90% homology considered), DNA sequence homology, and proportion | Microorganism or composition No. | Number of characteristic genes among the 12 gene sequences provided herein (only those with over 90% homology considered), DNA sequence homology, and proportion |
|---|---|---|---|
| Microbial inoculant 1 | 12: sequences 1-12, 100% homology for all | Microbial inoculant 2 | 12: sequences 1-12, 100% homology for all |
| | Proportion: 100% | | Proportion: 100% |
| Microbial inoculant 3 | 12: sequences 1-12, 100% homology for all | Microbial inoculant 4 | 12: sequences 1-12, 100% homology for all |
| | Proportion: 100% | | Proportion: 100% |
| Microbial inoculant 5 | 12: sequences 1-12, 100% homology for all | Microbial inoculant 6 | 12: sequences 1-12, 100% homology for all |
| | Proportion: 100% | | Proportion: 100% |
| Microbial inoculant 7 | 12: sequences 1-12, 100% homology for all | Microbial inoculant 8 | 12: sequences 1-12, 100% homology for all |
| | Proportion: 100% | | Proportion: 100% |
| Microbial inoculant 9 | 9: sequences 1-3 and 7-12, 100% homology for all | Microbial inoculant 10 | 9: sequences 1-3 and 7-12, 100% homology for all |
| | Proportion: 75% | | Proportion: 75% |
| Microbial inoculant 11 | 9: sequences 1-3 and 7-12, 100% homology for all | Microbial inoculant 12 | 9: sequences 1-3 and 7-12, 100% homology for all |
| | Proportion: 75% | | Proportion: 75% |
| Microbial inoculant 13 | 9: sequences 1-3 and 7-12, 100% homology for all | Microbial inoculant 14 | 9: sequences 1-3 and 7-12, 100% homology for all |
| | Proportion: 75% | | Proportion: 75% |
| Microbial inoculant 15 | 7: sequences 1-3 and 7-9, 100% homology for all; sequences 10 and 11, 90.3%-93.9% homology | Microbial inoculant 16 | 9: sequences 1-3 and 7-12, 100% homology for all |
| | Proportion: 67% | | Proportion: 75% |
| Microbial inoculant 17 | 9: sequences 1-3 and 7-12, 100% homology for all | Microbial inoculant 18 | 9: sequences 1-3 and 7-12, 100% homology for all |
| | Proportion: 75% | | Proportion: 75% |
| Microbial inoculant 19 | 9: sequences 1-3 and 7-12, 100% homology for all | Microbial inoculant 20 | 9: sequences 1-3 and 7-12, 100% homology for all |
| | Proportion: 75% | | Proportion: 75% |
| Microbial inoculant 21 | 9: sequences 1-3 and 7-12, 100% homology for all | Microbial inoculant 22 | 9: sequences 1-3 and 7-12, 100% homology for all |
| | Proportion: 75% | | Proportion: 75% |
| Microbial inoculant 23 | 9: sequences 1-3 and 7-12, 100% homology for all | Microbial inoculant 24 | 9: sequences 1-3 and 7-12, 100% homology for all |
| | Proportion: 75% | | Proportion: 75% |
| Microbial inoculant 25 | 9: sequences 1-3 and 7-12, 100% homology for all | Microbial inoculant 26 | 6: sequences 1-3 and 7-9, 90.1%-99.9% homology |
| | Proportion: 75% | | Proportion: 50% |
| Microbial inoculant 27 | 5: sequences 4-6, 8, and 9, 92.5%-99.6% homology | Microbial inoculant 28 | 5: sequences 1-3, 11, and 12, 90%-99.9% homology |
| | Proportion: 42% | | Proportion: 42% |
| Microbial inoculant 29 | 5: sequences 1-4 and 7, 91.2%-99.9% homology | Microbial inoculant 30 | 4: sequences 1, 2, 7, and 9, 90%-100% homology |
| | Proportion: 42% | | Proportion: 33% |
| Microbial inoculant 31 | 4: sequences 3, 4, 6, and 8, 90%-100% homology | Microbial inoculant 32 | 0, proportion: 0 |
| | Proportion: 33% | | |
| Microbial inoculant 33 | 0, proportion: 0 | Microbial inoculant 34 | 0, proportion: 0 |
| Microbial inoculant 35 | 0, proportion: 0 | Microbial inoculant 36 | 0, proportion: 0 |
| Microbial inoculant 37 | 0, proportion: 0 | Microbial inoculant 38 | 0, proportion: 0 |
| Microbial inoculant 39 | 0, proportion: 0 | Microbial inoculant 40 | 0, proportion: 0 |
| Microbial inoculant 41 | 4: sequences 4, 7, 10, and 12, 90%-99% homology | Microbial inoculant 42 | 3: sequences 3, 5, and 12, 96%-100% homology |
| | Proportion: 33% | | Proportion: 25% |

### Example 4: Determination of the Quality-Improving and Nitrogen-Fixing Coupled Effect of the Microbial Inoculant on Peanuts

Taking peanuts as an example, the steps for determining the quality-improving and nitrogen-fixing coupled effects of the above microbial inoculants are described as follows.

The microbial inoculants in Table 3 of Example 3 above were respectively applied to the field together with the basal fertilizer at peanut sowing, or could also be applied during the peanut growing period, with a cumulative amount of not less than 80 billion viable cells per mu of land. Plots without any of the above microbial inoculants were set as controls, and all other management followed conventional field practices. Continuous investigation was carried out after seedling emergence. Within the first 30 days after seedling emergence, early nodulation was investigated. At the flowering and pegging stage, the number of nodules, nodule weight, and nitrogenase activity per plant were investigated. From 1 to 15 days before harvest, the root nodulation status of the peanuts at the mature stage was investigated, i.e., whether the nodulation period was prolonged. From the seedling stage to the harvest stage, the alleviating effect on field diseases, i.e., the disease control effect, was investigated. After harvest, the total biomass, yield per unit area, and quality including protein, glutamic acid, and peanut resveratrol, as well as the abundance of *Aspergillus flavus* were investigated to determine whether there was an effect in improving quality and safety.

The investigation results are shown in Table 4. Based on the results in Table 4 above, the microbial inoculants 1-31 and 41 simultaneously possessed the following characteristics: 1) they contained at least 4 of the DNA sequences 1-12, and these genes might have not more than 10% base variations in different strains; 2) the microbial inoculants were capable of improving the quality of leguminous crop products by increasing the content of one or more nutritional functional components such as protein, glutamic acid, and peanut resveratrol; 3) the microbial inoculants had an alleviating effect, i.e., control effect, on one or more soil-borne diseases including pod rot in peanuts, stay-green syndrome in soybeans, and southern blight, bacterial wilt, root rot, and *Sclerotinia* disease in leguminous crops, or reduced spots on peanut pod surfaces, or significantly reduced aflatoxin levels and improved quality and safety; 4) although the microbial inoculants were not rhizobia, they were capable of simultaneously regulating and increasing the abundance of rhizobia in the rhizosphere of leguminous crops, increasing the number of nodules formed on the leguminous crops, and enhancing nitrogenase activity per plant; 5) the microbial inoculants were capable of promoting early nodulation of leguminous crops such as peanuts and soybeans and prolonging the nodulation and nitrogen fixation period; and 6) the microbial inoculants were capable of increasing the yield per unit area and total biomass of leguminous crops. In contrast, the microbial inoculants 32-40 and 42 could not simultaneously satisfy the above 6 characteristics.

**Table 4. Determination Results of the Quality-Improving and Nitrogen-Fixing Effect of the Microbial Inoculant on Peanuts**

| Microorganism or composition No. | Improved quality? | Control effect on diseases such as peanut pod rot, southern blight, and bacterial wilt, or reduced spots on peanut pod surfaces, or significantly reduced aflatoxin level and improved quality and safety? | Doubled promotion of nodulation and nitrogen fixation, early nodulation, prolonged nodulation and nitrogen fixation period, increased yield per unit area and total biomass? | Microorganism or composition No. | Improved quality or significantly improved quality and safety? | Control effect on diseases such as peanut pod rot, southern blight, and bacterial wilt, or reduced spots on peanut pod surfaces, or significantly reduced aflatoxin level and improved quality and safety? | Doubled promotion of nodulation and nitrogen fixation, early nodulation, prolonged nodulation and nitrogen fixation period, increased yield per unit area and total biomass? |
|---|---|---|---|---|---|---|---|
| Microbial inoculant 1 | Yes | Yes | Yes | Microbial inoculant 2 | Yes | Yes | Yes |
| Microbial inoculant 3 | Yes | Yes | Yes | Microbial inoculant 4 | Yes | Yes | Yes |
| Microbial inoculant 5 | Yes | Yes | Yes | Microbial inoculant 6 | Yes | Yes | Yes |
| Microbial inoculant 7 | Yes | Yes | Yes | Microbial inoculant 8 | Yes | Yes | Yes |
| Microbial inoculant 9 | Yes | Yes | Yes | Microbial inoculant 10 | Yes | Yes | Yes |
| Microbial inoculant 11 | Yes | Yes | Yes | Microbial inoculant 12 | Yes | Yes | Yes |
| Microbial inoculant 13 | Yes | Yes | Yes | Microbial inoculant 14 | Yes | Yes | Yes |
| Microbial inoculant 15 | Yes | Yes | Yes | Microbial inoculant 16 | Yes | Yes | Yes |
| Microbial inoculant 17 | Yes | Yes | Yes | Microbial inoculant 18 | Yes | Yes | Yes |
| Microbial inoculant 19 | Yes | Yes | Yes | Microbial inoculant 20 | Yes | Yes | Yes |
| Microbial inoculant 21 | Yes | Yes | Yes | Microbial inoculant 22 | Yes | Yes | Yes |
| Microbial inoculant 23 | Yes | Yes | Yes | Microbial inoculant 24 | Yes | Yes | Yes |
| Microbial inoculant 25 | Yes | Yes | Yes | Microbial inoculant 26 | Yes | Yes | Yes |
| Microbial inoculant 27 | Yes | Yes | Yes | Microbial inoculant 28 | Yes | Yes | Yes |
| Microbial inoculant 29 | Yes | Yes | Yes | Microbial inoculant 30 | Yes | Yes | Yes |
| Microbial inoculant 31 | Yes | Yes | Yes | Microbial inoculant 32 | No | No | No |
| Microbial inoculant 33 | No | No | No | Microbial inoculant 34 | No | No | No |
| Microbial inoculant 35 | No | No | No | Microbial inoculant 36 | No | No | No |
| Microbial inoculant 37 | No | No | No | Microbial inoculant 38 | No | No | No |
| Microbial inoculant 39 | No | No | No | Microbial inoculant 40 | No | No | No |
| Microbial inoculant 41 | Yes | Yes | Yes | Microbial inoculant 42 | No | No | No |

The above microbial inoculants were capable of improving the quality of leguminous crop products such as peanuts by increasing the content of one or more nutritional functional components such as protein, glutamic acid, and peanut resveratrol. Specifically, the improvements in peanut quality were evidenced by an increase of more than 0.2% (more preferably, over 3%) in protein content, a total increase of 0.15 mg/kg in essential amino acids for humans such as leucine (more preferably, over 0.2 mg/kg), and an increase of more than 0.2 mg/kg (more preferably, over 0.4 mg/kg) in resveratrol content.

### Example 5: Determination of the Quality-Improving and Nitrogen-Fixing Coupled Effect of the Microbial Inoculant on Soybeans

Taking soybeans as another example, the steps for determining the quality-improving and nitrogen-fixing coupled effects of the above microbial inoculants are described as follows.

The microbial inoculants in Table 3 of Example 3 above were respectively applied to the field together with the basal fertilizer at soybean sowing, or could also be applied during the soybean growing period, with a cumulative amount of not less than 80 billion viable cells per mu of land. Plots without any of the above microbial inoculants were set as controls, and all other management followed conventional field practices. Continuous investigation was carried out after seedling emergence. Within the first 30 days after seedling emergence, early nodulation was investigated. At the flowering and podding stage, the number of nodules, nodule weight, and nitrogenase activity per plant were investigated. From 1 to 15 days before harvest, the root nodulation status of the soybeans at the mature stage was investigated, i.e., whether the nodulation period was prolonged. From the seedling stage to the harvest stage, the alleviating effect on field diseases, i.e., the disease control effect, was investigated. After harvest, the total biomass, yield per unit area, and quality including protein, glutamic acid, and soybean isoflavone, as well as the abundance of *Aspergillus flavus* were investigated to determine whether there was an effect in improving quality and safety.

The above investigation results were substantially consistent with the results in Table 4. The above microbial inoculants were capable of improving the quality of leguminous crop products such as soybeans by increasing the content of one or more nutritional functional components such as protein, glutamic acid, and soybean isoflavone. Specifically, the improvements in soybean quality were evidenced by an increase of more than 0.3% (more preferably, over 3%) in protein content, a total increase of 0.2 mg/kg in essential amino acids for humans such as leucine (more preferably, over 0.3 mg/kg), and an increase of more than 50 mg/kg (more preferably, over 200 mg/kg) in soybean isoflavone.

Based on these results, the microbial inoculants 1-31 and 41 simultaneously possessed the following characteristics: 1) they contained at least 4 of the DNA sequences 1-12, and these genes might have not more than 10% base variations in different strains; 2) the microbial inoculants were capable of improving the quality of leguminous crop products by increasing the content of one or more nutritional functional components such as protein, glutamic acid, and soybean isoflavone; 3) the microbial inoculants had an alleviating effect, i.e., control effect, on one or more soil-borne diseases including pod rot in peanuts, stay-green syndrome in soybeans, and southern blight, bacterial wilt, root rot, and *Sclerotinia* disease in leguminous crops, or significantly reduced aflatoxin levels and improved quality and safety; 4) although the microbial inoculants were not rhizobia, they were capable of simultaneously regulating and increasing the abundance of rhizobia in the rhizosphere of leguminous crops, increasing the number of nodules formed on the leguminous crops, and enhancing nitrogenase activity per plant; 5) the microbial inoculants were capable of promoting early nodulation of leguminous crops such as peanuts and soybeans and prolonging the nodulation and nitrogen fixation period; and 6) the microbial inoculants were capable of increasing the yield per unit area and total biomass of leguminous crops. In contrast, the microbial inoculants 32-40 and 42 could not simultaneously satisfy the above 6 characteristics.

### Example 6: Determination of the Quality-Improving and Nitrogen-Fixing Coupled Effect of the Microbial Inoculant on Other Leguminous Crops

Using the same method and steps as those in Examples 4 and 5, the quality-improving and nitrogen-fixing coupled effects of the microbial inoculants on other leguminous crops such as peas, broad beans, cowpeas, and alfalfa were determined, and results similar to those for peanuts and soybeans above were obtained, with conclusions consistent with those listed in Table 4.

### Selection of the Quality-Improving and Nitrogen-Fixing Coupled Microbial Inoculant

Based on the results in Tables 1-4 above, the microbial inoculants 1-31 and 41 simultaneously possessed the following characteristics: 1) they contained at least 4 of the DNA sequences 1-12, and these genes might have not more than 10% base variations in different strains; 2) the microbial inoculants were capable of improving the quality of leguminous crop products by increasing the content of one or more nutritional functional components such as protein, glutamic acid, peanut resveratrol, and soybean isoflavone; 3) the microbial inoculants had an alleviating effect, i.e., control effect, on one or more soil-borne diseases including pod rot in peanuts, stay-green syndrome in soybeans, and southern blight, bacterial wilt, root rot, and *Sclerotinia* disease in leguminous crops, or reduced spots on peanut pod surfaces, or significantly reduced aflatoxin levels and improved quality and safety; 4) although the microbial inoculants were not rhizobia, they were capable of simultaneously regulating and increasing the abundance of rhizobia in the rhizosphere of leguminous crops, increasing the number of nodules formed on the leguminous crops, and thereby enhancing nitrogenase activity per plant; 5) the microbial inoculants were capable of promoting early nodulation of leguminous crops such as peanuts and soybeans and prolonging the nodulation and nitrogen fixation period; and 6) the microbial inoculants were capable of increasing the yield per unit area and total biomass of leguminous crops.

Knockout experiments were performed on the microbial inoculant of this disclosure with respect to DNA sequences 1-12. The results showed that when 4 or more gene sequences remained after knockout, the microbial inoculant still retained not only the quality-improving and nitrogen-fixing coupling effect of this disclosure to improve quality of leguminous crop products but also the effects of regulating and increasing abundance of rhizobia in rhizosphere of leguminous crops and increasing the number of nodules formed on the leguminous crops, as well as the coupling effect of further promoting early nodulation and prolonging the nodulation and nitrogen fixation period of the leguminous crops. When only 3 or fewer gene sequences remained after knockout, the microbial inoculant no longer simultaneously possessed the quality-improving and nitrogen-fixing coupling effect, or the effects of regulating and increasing abundance of rhizobia in rhizosphere of leguminous crops and increasing the number of nodules formed on the leguminous crops, or the coupling effect of further promoting early nodulation and prolonging the nodulation and nitrogen fixation period of the leguminous crops.

### Example 7: Use of the Quality-Improving and Nitrogen-Fixing Coupled Microbial Inoculant - Improving Quality and Safety of Leguminous Crop Products

**Taking the above microbial inoculants 1, 7, and 25 as examples, the use of the quality-improving and nitrogen-fixing coupled microbial inoculant in improving quality and safety of leguminous crop products is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After the above peanuts were harvested, peanut samples were collected by national standard sampling methods: (1) the abundance of aflatoxin-producing fungi carried by these samples was determined by the classical colony counting method, and the reduction rate of the abundance of aflatoxin-producing fungi carried by the peanuts, i.e., the control effect against aflatoxin-producing fungi, was calculated; (2) the contents of peanut resveratrol, protein, and leucine were determined using standard methods, and the level of quality improvement was calculated; and (3) after the samples were placed under the same conditions for 6 months, the aflatoxin contamination level was determined by the national standard liquid chromatography-mass spectrometry method, and the control effect against aflatoxins was calculated. The determination results showed that the microbial inoculants 1, 7, and 25 all achieved a control effect of more than 62% against aflatoxin-producing fungi carried by field-grown peanut pods, and a control effect of more than 80% against aflatoxins in peanuts; the protein content was increased by over 0.2%; the total content of essential amino acids for humans such as leucine was increased by 0.15 mg/kg; and the peanut resveratrol content was increased by over 0.2 mg/kg. The above results indicate that the use of the quality-improving and nitrogen-fixing coupled microbial inoculant can significantly improve the quality and safety of field-grown peanuts.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. After harvest, soybean samples were collected using national standard sampling methods, and the contents of soybean isoflavone, protein, and amino acids were determined using standard methods, and the level of quality improvement was calculated. The determination results showed that the use of the microbial inoculants 1, 7, and 25 significantly increased the contents of soybean isoflavone, protein, and glutamic acid in the field-grown soybeans; the protein content was increased by over 0.3%; the total content of essential amino acids for humans such as leucine was increased by over 0.2 mg/kg; and the soybean isoflavone content was increased by over 50 mg/kg. The above results indicate that the use of the quality-improving and nitrogen-fixing coupled microbial inoculant can significantly improve the quality of field-grown soybeans.
3. Use in other leguminous crops: it also achieved results similar to those obtained for soybeans described above.

### Example 8: Use of the Quality-Improving and Nitrogen-Fixing Coupled Microbial Inoculant - Promoting Nodulation and Nitrogen Fixation of Leguminous Crops

**Taking the above microbial inoculants 1, 7, and 25 as examples, the use of the quality-improving and nitrogen-fixing coupled microbial inoculant in promoting nodulation and nitrogen fixation of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, peanut root nodulation was continuously investigated. The investigation results showed that at the flowering stage, the number of nodules formed on peanut roots treated with the microbial inoculants 1, 7, and 25 all increased by over 2.3 times, and the nitrogenase activity per plant increased by over 7.6 times. The above results indicate that the use of the quality-improving and nitrogen-fixing coupled microbial inoculant can significantly promote the nodulation and nitrogen fixation level of field-grown peanuts.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. After seedling emergence, soybean root nodulation was continuously investigated. The investigation results showed that at the flowering stage, the number of nodules formed on soybean roots treated with the microbial inoculants 1, 7, and 25 all increased by over 2.3 times, and the nitrogenase activity per plant increased by over 6.9 times. The above results indicate that the use of the quality-improving and nitrogen-fixing coupled microbial inoculant can significantly promote the nodulation and nitrogen fixation level of field-grown soybeans.
3. Use in other leguminous crops: it also achieved results similar to those obtained for peanuts and soybeans described above in terms of promoting nodulation and nitrogen fixation.

### Example 9: Use of the Quality-Improving and Nitrogen-Fixing Coupled Microbial Inoculant - Increasing the Yield per Unit Area of Leguminous Crops

**Taking the above microbial inoculants 1, 7, and 25 as examples, the use of the quality-improving and nitrogen-fixing coupled microbial inoculant in increasing the yield per unit area of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At harvest, actual yields were measured from one-mu plots for both the microbial inoculant treatment groups and the control group. Calculations showed that the peanut yield enhancement rates of the plots treated with the microbial inoculants 1, 7, and 25 were all above 12%, reaching a significant yield enhancement level. The above results indicate that the use of the quality-improving and nitrogen-fixing coupled microbial inoculant can significantly increase the yield per unit area of field-grown peanuts.
2. Use in soybeans: the above microbial inoculants 1, 7, and 25 were respectively applied uniformly to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. At harvest, actual yields were measured from one-mu plots for both the microbial inoculant treatment groups and the control group. Calculations showed that the soybean yield enhancement rates of the plots treated with the microbial inoculants 1, 7, and 25 were all above 7%, reaching a significant yield enhancement level. The above results indicate that the use of the quality-improving and nitrogen-fixing coupled microbial inoculant can significantly increase the yield per unit area of field-grown soybeans.
3. Use in peas: the above microbial inoculants 1, 7, and 25 were respectively applied uniformly to the field by manual broadcasting before pea sowing, with a cumulative amount of the microbial inoculants of 80 to 100 billion viable cells per mu of land. Plots without any of the above microbial inoculants were set as controls, and all other management followed conventional field practices. At the time of harvesting pea shoots and green pea pods, respectively, the yields were measured. The yield enhancement rates of pea shoots and pea pods treated with the microbial inoculants 1, 7, and 25 were all above 5%, reaching a significant yield enhancement level. The above results indicate that the use of the quality-improving and nitrogen-fixing coupled microbial inoculant can significantly increase the yield per unit area of field-grown pea shoots and pea pods.
4. Use in other leguminous crops: the use of the microbial inoculants 1, 7, and 25 in leguminous crops such as broad beans achieved results similar to those obtained for soybeans described above. The use of the microbial inoculants 1, 7, and 25 in common beans and cowpeas achieved results similar to those obtained for peas described above. The use of the microbial inoculants 1, 7, and 25 in alfalfa achieved results similar to those obtained for peas described above.

### Example 10: Use of the Quality-Improving and Nitrogen-Fixing Coupled Microbial Inoculant - Recruiting Indigenous Rhizobia and Increasing the Abundance of Rhizobia in the Rhizosphere Soil of Leguminous Crops

**Taking the above microbial inoculants 1, 7, and 25 as examples, the use of the quality-improving and nitrogen-fixing coupled microbial inoculant in increasing the Abundance of Rhizobia of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At the pod-filling stage and before harvest, peanut rhizosphere soil samples were collected, and metagenomic sequencing and big data analysis were performed. The investigation results showed that the abundance of rhizobia in the rhizosphere soil of peanuts treated with the microbial inoculants 1, 7, and 25 was significantly higher than the control group, with an increase of 10.3%-27.1%. The above results indicate that the use of the quality-improving and nitrogen-fixing coupled microbial inoculant has a significant effect of recruiting indigenous rhizobia and thereby increasing the abundance of rhizobia in the rhizosphere soil of leguminous crops.
2. Use in other leguminous crops such as soybeans, peas, broad beans, cowpeas, and alfalfa: it also achieved results similar to those obtained for peanuts described above in terms of recruiting indigenous rhizobia and increasing the abundance of rhizobia in the rhizosphere soil of leguminous crops.

### Example 11: Use of the Quality-Improving and Nitrogen-Fixing Coupled Microbial Inoculant - Promoting Earlier Nodulation and Prolonging the Nodulation and Nitrogen Fixation Period of Leguminous Crops

**Taking the above microbial inoculants 1, 7, and 25 as examples, the use of the quality-improving and nitrogen-fixing coupled microbial inoculant in promoting earlier nodulation and prolonging the nodulation and nitrogen fixation period of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, peanut root nodulation was continuously investigated. The investigation results showed that in the peanuts treated with the microbial inoculants 1, 7, and 25, nodulation on roots occurred as early as about 7-9 days after seedling emergence, which was more than 20 days earlier than the conventional condition of nodulation beginning around 30 days after seedling emergence; at the pod-filling stage, newly formed nodules could still be found, presenting a scene where senescent nodules, mature nodules, and young nodules coexisted; at harvest, fresh nodules with nitrogenase activity could still be found, breaking the conventional understanding that no new nodules are formed after the pod-filling stage and that all nodules senesce by harvest, significantly prolonging the nodulation and nitrogen fixation period. The above results indicate that the use of the quality-improving and nitrogen-fixing coupled microbial inoculant can promote earlier nodulation and prolong the nodulation and nitrogen fixation period of leguminous crops such as peanuts.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. After seedling emergence, soybean root nodulation was continuously investigated. The investigation results showed that in the soybeans treated with the microbial inoculants 1, 7, and 25, nodulation on roots occurred as early as about 6-8 days after seedling emergence, which was about 20 days earlier than the conventional condition of nodulation beginning around 30 days after seedling emergence; at the pod-filling stage, newly formed nodules could still be found, presenting a scene where senescent nodules, mature nodules, and young nodules coexisted; at harvest, fresh nodules with nitrogenase activity could still be found, breaking the conventional understanding that no new nodules are formed after the pod-filling stage and that all nodules senesce by harvest, significantly prolonging the nodulation and nitrogen fixation period. The above results indicate that the use of the quality-improving and nitrogen-fixing coupled microbial inoculant can promote earlier nodulation and prolong the nodulation and nitrogen fixation period.
3. Use in other leguminous crops: the above microbial inoculants 1, 7, and 25 respectively achieved results similar to those obtained for peanuts and soybeans described above in terms of promoting earlier nodulation and prolonging the nodulation and nitrogen fixation period of leguminous crops.

### Example 12: Use of the Quality-Improving and Nitrogen-Fixing Coupled Microbial Inoculant - Preventing Nutrient Deficiency and Premature Senescence of Leguminous Crops at the Maturity Stage

**Taking the above microbial inoculants 1, 7, and 25 as examples, the use of the quality-improving and nitrogen-fixing coupled microbial inoculant in preventing nutrient deficiency and premature senescence of leguminous crops at the maturity stage is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At the maturity stage, the leaf photosynthetic rates and senescence status of peanuts were continuously investigated. The investigation results showed that in the peanuts treated with the microbial inoculants 1, 7, and 25, the leaf photosynthetic rates were all increased by over 33% compared with the control group, exhibiting a significant effect in preventing nutrient deficiency and premature senescence. The above results indicate that the use of the quality-improving and nitrogen-fixing coupled microbial inoculant can effectively prevent nutrient deficiency and premature senescence of leguminous crops at the maturity stage.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. After seedling emergence, soybean root nodulation was continuously investigated. The investigation results showed that in the soybeans treated with the microbial inoculants 1, 7, and 25, the leaf photosynthetic rates were all increased by over 23.5%, exhibiting a significant effect in preventing nutrient deficiency and premature senescence. The above results indicate that the use of the quality-improving and nitrogen-fixing coupled microbial inoculant can promote earlier nodulation and prolong the nodulation and nitrogen fixation period.
3. Use in other leguminous crops: the use of the microbial inoculants 1, 7, and 25 in leguminous crops such as peas, broad beans, and cowpeas achieved results similar to those obtained for peanuts and soybeans described above in terms of preventing nutrient deficiency and premature senescence at the maturity stage.

### Example 13: Use of the Quality-Improving and Nitrogen-Fixing Coupled Microbial Inoculant - Increasing the Number of Pods of Leguminous Crops

### Taking the above microbial inoculants 1, 7, and 25 as examples, the use of the quality-improving and nitrogen-fixing coupled microbial inoculant in increasing the number of pods of leguminous crops is described below.

1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At harvest, the number of peanut pods was investigated. The investigation results showed that in the peanuts treated with the microbial inoculants 1, 7, and 25, the number of effective pods was all increased by over 16.3% compared with the control group, achieving a significant effect in increasing the number of pods. The above results indicate that the use of the quality-improving and nitrogen-fixing coupled microbial inoculant can significantly increase the number of peanut pods.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. At harvest, the number of soybean pods was investigated. The investigation results showed that in the soybeans treated with the microbial inoculants 1, 7, and 25, the number of pods was all increased by over 13.5%, achieving a significant effect in increasing the number of pods. The above results indicate that the use of the quality-improving and nitrogen-fixing coupled microbial inoculant can significantly increase the number of soybean pods.
3. Use in other leguminous crops such as peas, broad beans, common beans, and cowpeas: it also achieved results similar to those obtained for peanuts and soybeans described above in terms of significantly increasing the number of pods.

### Example 14: Use of the Quality-Improving and Nitrogen-Fixing Coupled Microbial Inoculant - Increasing the Fullness of Pods and Reducing the Rate of Shriveled Pods of Leguminous Crops

**Taking the above microbial inoculants 1, 7, and 25 as examples, the use of the quality-improving and nitrogen-fixing coupled microbial inoculant in increasing the fullness of pods and reducing the rate of shriveled pods of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At harvest, the condition of peanut pods was investigated and the rate of shriveled pods was calculated. The investigation results showed that in the peanuts treated with the microbial inoculants 1, 7, and 25, the fullness of pods was significantly improved, and the rate of shriveled pods was reduced by over 7.5%. The above results indicate that the use of the quality-improving and nitrogen-fixing coupled microbial inoculant has a significant effect of increasing the fullness of peanut pods and reducing the rate of shriveled pods.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. At harvest, the condition of soybean pods was investigated and the rate of shriveled pods was calculated. The investigation results showed that in the soybeans treated with the microbial inoculants 1, 7, and 25, the fullness of pods was significantly improved, and the rate of shriveled pods was reduced by over 5.7%. The above results indicate that the use of the quality-improving and nitrogen-fixing coupled microbial inoculant has a significant effect of increasing the fullness of soybean pods and reducing the rate of shriveled pods.
3. Use in other leguminous crops such as peas, broad beans, common beans, and cowpeas: the microbial inoculants 1, 7, and 25 also achieved results similar to those obtained for peanuts and soybeans described above in terms of increasing the fullness of pods and reducing the rate of shriveled pods.

### Example 15: Use of the Quality-Improving and Nitrogen-Fixing Coupled Microbial Inoculant - Promoting Earlier Flowering and Earlier Podding of Leguminous Crops

**Taking the above microbial inoculants 1, 7, and 25 as examples, the use of the quality-improving and nitrogen-fixing coupled microbial inoculant in promoting earlier flowering and earlier podding of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the peanut flowering and podding time of the treatment and control groups. The investigation results showed that in the peanuts treated with the microbial inoculants 1, 7, and 25, flowering and podding both occurred at least 2 days earlier. The above results indicate that the use of the quality-improving and nitrogen-fixing coupled microbial inoculant has a significant effect of promoting earlier flowering and earlier podding in peanuts.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the soybean flowering and podding time of the treatment and control groups. The investigation results showed that in the soybeans treated with the microbial inoculants 1, 7, and 25, flowering and podding both occurred at least 2 days earlier. The above results indicate that the use of the quality-improving and nitrogen-fixing coupled microbial inoculant has a significant effect of promoting earlier flowering and earlier podding in soybeans.
3. Use in other leguminous crops such as peas, broad beans, common beans, and cowpeas: it also achieved results similar to those obtained for peanuts and soybeans described above in terms of promoting earlier flowering and earlier podding.

### Example 16: Use of the Quality-Improving and Nitrogen-Fixing Coupled Microbial Inoculant - Reducing the Occurrence of Pod Rot in Peanuts

**Taking the above microbial inoculants 1, 7, and 25 as examples, the use of the quality-improving and nitrogen-fixing coupled microbial inoculant in reducing the severity of pod rot in peanuts is described below.**

The above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At harvest, the severity of pod rot in peanuts in the treatment and control groups was recorded. The investigation results showed that in the peanuts treated with the microbial inoculants 1, 7, and 25, the severity of pod rot was reduced by over 60% compared with the control group. The above results indicate that the use of the quality-improving and nitrogen-fixing coupled microbial inoculant has a significant effect of reducing pod rot in peanuts.

### Example 17: Use of the Quality-Improving and Nitrogen-Fixing Coupled Microbial Inoculant - Reducing the Occurrence of Bacterial Wilt in Leguminous Crops

**Taking the above microbial inoculants 1, 7, and 25 as examples, the use of the quality-improving and nitrogen-fixing coupled microbial inoculant in reducing the severity of bacterial wilt in leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of bacterial wilt in the peanuts in the treatment and control groups. The investigation results showed that in the peanuts treated with the microbial inoculants 1, 7, and 25, the severity of bacterial wilt was reduced by over 55% compared with the control group. The above results indicate that the use of the quality-improving and nitrogen-fixing coupled microbial inoculant has a significant effect of reducing bacterial wilt in peanuts.
2. Use in other leguminous crops such as soybeans, peas, broad beans, common beans, and cowpeas: it also achieved results similar to those obtained for peanuts described above in terms of reducing the severity of bacterial wilt in leguminous crops.

### Example 18: Use of the Quality-Improving and Nitrogen-Fixing Coupled Microbial Inoculant - Reducing the Occurrence of Powdery Mildew in Leguminous Crops

**Taking the above microbial inoculants 1, 7, and 25 as examples, the use of the quality-improving and nitrogen-fixing coupled microbial inoculant in reducing the severity of powdery mildew in leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of powdery mildew in the peanuts in the treatment and control groups. The investigation results showed that in the peanuts treated with the microbial inoculants 1, 7, and 25, the severity of powdery mildew was reduced by over 60% compared with the control group. The above results indicate that the use of the quality-improving and nitrogen-fixing coupled microbial inoculant has a significant effect of reducing powdery mildew in peanuts.
2. Use in other leguminous crops such as soybeans, peas, broad beans, common beans, and cowpeas: it also achieved results similar to those obtained for peanuts described above in terms of reducing the severity of powdery mildew in leguminous crops.

### Example 19: Use of the Quality-Improving and Nitrogen-Fixing Coupled Microbial Inoculant - Reducing the Occurrence of Leaf Spot in Leguminous Crops

**Taking the above microbial inoculants 1, 7, and 25 as examples, the use of the quality-improving and nitrogen-fixing coupled microbial inoculant in reducing the severity of leaf spot in leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of leaf spot in the peanuts in the treatment and control groups. The investigation results showed that in the peanuts treated with the microbial inoculants 1, 7, and 25, the severity of leaf spot was reduced by over 53% compared with the control group. The above results indicate that the use of the quality-improving and nitrogen-fixing coupled microbial inoculant has a significant effect of reducing leaf spot in peanuts.
2. Use in other leguminous crops such as soybeans, peas, broad beans, common beans, and cowpeas: it also achieved results similar to those obtained for peanuts described above in terms of reducing the severity of leaf spot in leguminous crops.

### Example 20: Use of the Quality-Improving and Nitrogen-Fixing Coupled Microbial Inoculant - Reducing the Occurrence of Root-Knot Nematode Disease in Leguminous Crops

**Taking the above microbial inoculants 1, 7, and 25 as examples, the use of the quality-improving and nitrogen-fixing coupled microbial inoculant in reducing the severity of root-knot nematode disease in leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of root-knot nematode disease in the peanuts in the treatment and control groups. The investigation results showed that in the peanuts treated with the microbial inoculants 1, 7, and 25, the severity of root-knot nematode disease was reduced by over 60% compared with the control group. The above results indicate that the use of the quality-improving and nitrogen-fixing coupled microbial inoculant has a significant effect of reducing root-knot nematode disease in peanuts.
2. Use in other leguminous crops such as soybeans, peas, broad beans, common beans, and cowpeas: it also achieved results similar to those obtained for peanuts described above in terms of reducing the severity of root-knot nematode disease in leguminous crops.

### Example 21: Use of the Quality-Improving and Nitrogen-Fixing Coupled Microbial Inoculant - Reducing the Occurrence of Root Rot in Leguminous Crops

**Taking the above microbial inoculants 1, 7, and 25 as examples, the use of the quality-improving and nitrogen-fixing coupled microbial inoculant in reducing the severity of root rot in leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of root rot in the peanuts in the treatment and control groups. The investigation results showed that in the peanuts treated with the microbial inoculants 1, 7, and 25, the severity of root rot was reduced by over 57.5% compared with the control group. The above results indicate that the use of the quality-improving and nitrogen-fixing coupled microbial inoculant has a significant effect of reducing root rot in peanuts.
2. Use in soybeans: the above microbial inoculants 1, 7, and 25 were respectively mixed with the basal fertilizer at soybean sowing and applied to the field by unmanned aerial vehicles, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of root rot in the soybeans in the treatment and control groups. The investigation results showed that in the soybeans treated with the microbial inoculants 1, 7, and 25, the severity of root rot was reduced by over 55% compared with the control group. The above results indicate that the use of the quality-improving and nitrogen-fixing coupled microbial inoculant has a significant effect of reducing root rot in soybeans.
3. Use in other leguminous crops such as peas, broad beans, common beans, and cowpeas: it also achieved results similar to those obtained for peanuts and soybeans described above in terms of reducing root rot.

### Example 22: Use of the Quality-Improving and Nitrogen-Fixing Coupled Microbial Inoculant - Reducing the Occurrence of Sclerotinia Disease in Leguminous Crops

**Taking the above microbial inoculants 1, 7, and 25 as examples, the use of the quality-improving and nitrogen-fixing coupled microbial inoculant in reducing the severity of** *Sclerotinia* **disease in leguminous crops is described below.**
1. Use in soybeans: the above microbial inoculants 1, 7, and 25 were respectively mixed with the basal fertilizer at soybean sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of *Sclerotinia* disease in the soybeans in the treatment and control groups. The investigation results showed that in the soybeans treated with the microbial inoculants 1, 7, and 25, the severity of *Sclerotinia* disease was reduced by over 62% compared with the control group. The above results indicate that the use of the quality-improving and nitrogen-fixing coupled microbial inoculant has a significant effect of reducing *Sclerotinia* disease in soybeans.
2. Use in other leguminous crops such as peanuts, peas, broad beans, common beans, and cowpeas: it also achieved results similar to those obtained for soybeans described above in terms of reducing the severity of *Sclerotinia* disease in leguminous crops.

### Example 23: Use of the Quality-Improving and Nitrogen-Fixing Coupled Microbial Inoculant - Reducing the Occurrence of Downy Mildew in Leguminous Crops

**Taking the above microbial inoculants 1, 7, and 25 as examples, the use of the quality-improving and nitrogen-fixing coupled microbial inoculant in reducing the severity of downy mildew in leguminous crops is described below.**
1. Use in soybeans: the above microbial inoculants 1, 7, and 25 were respectively mixed with the basal fertilizer at soybean sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of downy mildew in the soybeans in the treatment and control groups. The investigation results showed that in the soybeans treated with the microbial inoculants 1, 7, and 25, the severity of downy mildew was reduced by over 58% compared with the control group. The above results indicate that the use of the quality-improving and nitrogen-fixing coupled microbial inoculant has a significant effect of reducing downy mildew in soybeans.
2. Use in other leguminous crops such as peanuts, peas, broad beans, common beans, and cowpeas: it also achieved results similar to those obtained for soybeans described above in terms of reducing the severity of downy mildew in leguminous crops.

### Example 24: Use of the Quality-Improving and Nitrogen-Fixing Coupled Microbial Inoculant - Reducing the Occurrence of Fusarium Wilt in Leguminous Crops

**Taking the above microbial inoculants 1, 7, and 25 as examples, the use of the quality-improving and nitrogen-fixing coupled microbial inoculant in reducing the severity of *Fusarium* wilt in leguminous crops is described below.**
1. Use in soybeans: the above microbial inoculants 1, 7, and 25 were respectively mixed with the basal fertilizer at soybean sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of ***Fusarium*** wilt in the soybeans in the treatment and control groups. The investigation results showed that in the soybeans treated with the microbial inoculants 1, 7, and 25, the severity of ***Fusarium*** wilt was reduced by nearly 60% compared with the control group. The above results indicate that the use of the quality-improving and nitrogen-fixing coupled microbial inoculant has a significant effect of reducing *Fusarium* wilt in soybeans.
2. Use in other leguminous crops such as peanuts, peas, broad beans, common beans, and cowpeas: it also achieved results similar to those obtained for soybeans described above in terms of reducing the severity of *Fusarium* wilt in leguminous crops.

### Example 25: Use of the Quality-Improving and Nitrogen-Fixing Coupled Microbial Inoculant - Reducing the Occurrence of Southern Blight in Leguminous Crops

**Taking the above microbial inoculants 1, 7, and 25 as examples, the use of the quality-improving and nitrogen-fixing coupled microbial inoculant in reducing the severity of southern blight in leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence continuous investigation was carried out to record the severity of southern blight in the peanuts in the treatment and control groups. The investigation results showed that in the peanuts treated with the microbial inoculants 1, 7, and 25, the severity of southern blight was reduced by over 60% compared with the control group. The above results indicate that the use of the quality-improving and nitrogen-fixing coupled microbial inoculant has a significant effect of reducing southern blight in peanuts.
2. Use in other leguminous crops such as soybeans, peas, broad beans, common beans, and cowpeas: it also achieved results similar to those obtained for peanuts described above in terms of reducing the severity of southern blight in leguminous crops.

### Example 26: Use of the Quality-Improving and Nitrogen-Fixing Coupled Microbial Inoculant - Reducing the Severity of Stay-Green Syndrome in Soybeans

**Taking the above microbial inoculants 1, 7, and 25 as examples, the use of the quality-improving and nitrogen-fixing coupled microbial inoculant in reducing the severity of stay-green syndrome in soybeans is described below.**

The above microbial inoculants 1, 7, and 25 were respectively mixed with the basal fertilizer at soybean sowing and applied to the field through a seeder, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the severity of stay-green syndrome in the soybeans in the treatment and control groups. The investigation results showed that in the soybeans treated with the microbial inoculants 1, 7, and 25, the severity of stay-green syndrome was reduced by over 40% compared with the control group. The above results indicate that the use of the quality-improving and nitrogen-fixing coupled microbial inoculant has a significant effect of reducing the severity of stay-green syndrome in soybeans.

### Example 27: Use of the Quality-Improving and Nitrogen-Fixing Coupled Microbial Inoculant - Promoting Carbon Emission Reduction in Leguminous Crops and Facilitating Soil Improvement

**Taking the above microbial inoculants 1, 7, and 25 as examples, the use of the quality-improving and nitrogen-fixing coupled microbial inoculant in promoting carbon emission reduction in leguminous crops is described below.**
1. Use in peanuts: the above microbial inoculants 1, 7, and 25 were respectively mixed with the basal fertilizer at peanut sowing and applied to the field through a seeder, where the nitrogen application rates in the basal fertilizer were reduced by 20% and 30%, respectively, compared to the conventional rate used in the control group, with a cumulative amount of the microbial inoculants of 80 to 100 billion viable cells per mu of land. Plots without any of the above microbial inoculants were set as controls, and all other management followed conventional field practices. After seedling emergence, continuous investigation was carried out to record the carbon emission reduction level in the treatment and control groups. The investigation results showed that in the peanuts treated with the microbial inoculants 1, 7, and 25, carbon dioxide emissions were reduced by 20%-30%, while also achieving significant yield enhancement of about 10%. The above results indicate that the use of the quality-improving and nitrogen-fixing coupled microbial inoculant has a significant effect of promoting carbon emission reduction during peanut production.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land, where the treatment groups had nitrogen fertilizer application reduced by 20% or 50% compared with the conventional fertilization level in the control group. Plots without any of the above microbial inoculants were set as controls, and all other management followed conventional field practices. After seedling emergence, continuous investigation was carried out to record the carbon emission reduction level in the treatment and control groups. The investigation results showed that in the soybeans treated with the microbial inoculants 1, 7, and 25, carbon dioxide emissions were reduced by 20%-50%, while also achieving significant yield enhancement of more than 5%. The above results indicate that the use of the quality-improving and nitrogen-fixing coupled microbial inoculant has a significant effect of promoting carbon emission reduction during soybean production.
3. Use in other leguminous crops such as peas, broad beans, common beans, and cowpeas: it also achieved results similar to those obtained for peanuts and soybeans described above in terms of promoting carbon emission reduction.

### Example 28: Use of the Quality-Improving and Nitrogen-Fixing Coupled Microbial Inoculant - Promoting an Increase in Total Biomass of Leguminous Crops

**Taking the above microbial inoculants 1, 7, and 25 as examples, the use of the quality-improving and nitrogen-fixing coupled microbial inoculant in promoting an increase in total biomass of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At harvest, investigation was carried out to record the total biomass of plants in the treatment and control groups. The investigation results showed that in the peanuts treated with the microbial inoculants 1, 7, and 25, the total biomass of plants was increased by over 22%. The above results indicate that the use of the quality-improving and nitrogen-fixing coupled microbial inoculant has a significant effect of promoting an increase in total biomass of peanut plants.
2. Use in soybeans: the above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. At maturity, investigation was carried out to record the total biomass of plants in the treatment and control groups. The investigation results showed that in the soybeans treated with the microbial inoculants 1, 7, and 25, the total biomass of plants was increased by over 13%. The above results indicate that the use of the quality-improving and nitrogen-fixing coupled microbial inoculant has a significant effect of promoting an increase in total biomass of soybean plants.
3. Use in other leguminous crops such as peas, broad beans, common beans, and cowpeas: it also achieved results similar to those obtained for peanuts and soybeans described above in terms of promoting an increase in total biomass of plants of leguminous crops.

### Example 29: Use of the Quality-Improving and Nitrogen-Fixing Coupled Microbial Inoculant - Reducing the Abundance of Harmful Organisms Such as Aspergillus and Fusarium in the Rhizosphere Soil of Leguminous Crops, and Facilitating Improvement of Soil Microbial Community Structure

**Taking the above microbial inoculants 1, 7, and 25 as examples, the use of the quality-improving and nitrogen-fixing coupled microbial inoculant in reducing the abundance of harmful organisms such as *Aspergillus* and *Fusarium* in the rhizosphere soil of leguminous crops is described below.**
1. Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At harvest, rhizosphere soil samples were collected from the peanuts, and the abundance of harmful organisms such as *Aspergillus* and ***Fusarium*** in the rhizosphere soil samples from the treatment and control groups was determined by metagenomic sequencing and big data analysis. The investigation results showed that in the peanuts treated with the microbial inoculants 1, 7, and 25, the abundance of *Aspergillus* and *Fusarium* in the rhizosphere soil was reduced by 45%-70%. The above results indicate that the use of the quality-improving and nitrogen-fixing coupled microbial inoculant has a significant effect of reducing the abundance of harmful organisms such as *Aspergillus* and ***Fusarium*** in the rhizosphere soil of leguminous crops.
2. Use in soybeans: the above microbial inoculants 1, 7, and 25 were respectively mixed with the basal fertilizer at soybean sowing and applied to the field by unmanned aerial vehicles, with a cumulative amount of the microbial inoculants of 80 to 100 billion viable cells per mu of land. Plots without any of the above microbial inoculants were set as controls, and all other management followed conventional field practices. At harvest, rhizosphere soil samples were collected from the soybeans, and the abundance of harmful organisms such as *Aspergillus* and *Fusarium* in the rhizosphere soil samples from the treatment and control groups was determined by metagenomic sequencing and big data analysis. The investigation results showed that in the soybeans treated with the microbial inoculants 1, 7, and 25, the abundance of *Aspergillus* and *Fusarium* in the rhizosphere soil was reduced by 45%-70%. The above results indicate that the use of the quality-improving and nitrogen-fixing coupled microbial inoculant has a significant effect of reducing the abundance of harmful organisms such as *Aspergillus* and ***Fusarium*** in the rhizosphere soil of leguminous crops.
3. Use in other leguminous crops such as peas, broad beans, common beans, and cowpeas: it also achieved results similar to those obtained for peanuts and soybeans described above in terms of significantly reducing the abundance of harmful organisms such as *Aspergillus* and ***Fusarium*** in the rhizosphere soil of leguminous crops.

### Example 30: Use of the Quality-Improving and Nitrogen-Fixing Coupled Microbial Inoculant - Reducing Spots on Peanut Pod Surfaces and Improving Marketability

**Taking the above microbial inoculants 1, 7, and 25 as examples, the use of the quality-improving and nitrogen-fixing coupled microbial inoculant in reducing spots on peanut pod surfaces is described below.**

Use in peanuts: the above microbial inoculants 1, 7, and 25 were respectively mixed with the basal fertilizer at peanut sowing and applied to the field through a seeder, with a cumulative amount of the microbial inoculants of 80 to 100 billion viable cells per mu of land. Plots without any of the above microbial inoculants were set as controls, and all other management followed conventional field practices. At harvest, the black spots on peanut pod surfaces in the treatment and control groups were investigated and recorded. The investigation results showed that in the peanuts treated with the microbial inoculants 1, 7, and 25, the black spots on peanut pod surfaces were reduced by over 70%. The above results indicate that the use of the quality-improving and nitrogen-fixing coupled microbial inoculant has a significant effect of reducing spots on peanut pod surfaces, improving marketability and competitiveness, and increasing economic benefits.

### Example 31: Use of the Quality-Improving and Nitrogen-Fixing Coupled Microbial Inoculant - Promoting Crop Resistance to Continuous Cropping Obstacles

**Continuous cropping of crops for many consecutive years often leads to the accumulation of pathogenic microorganisms, resulting in weak seedlings, diseased seedlings, and missing plants. Taking the above microbial inoculants 1, 7, and 25 as examples, the use of the quality-improving and nitrogen-fixing coupled microbial inoculant in promoting crop resistance to continuous cropping obstacles is described below.**
1. Use in peanuts: fields with many years of continuous peanut cultivation in Xingcheng City, Liaoning Province were selected to conduct demonstration trials on crop resistance to continuous cropping obstacles. The above microbial inoculants 1, 7, and 25 were respectively mixed with the basal fertilizer at peanut sowing and applied to the continuous cropping fields through a seeder, with a cumulative amount of the microbial inoculants of 80 to 100 billion viable cells per mu of land. Plots without any of the above microbial inoculants were set as controls, and all other management followed conventional field practices. After sowing, irregular and continuous investigations were carried out on the peanuts. The investigation results showed that in the peanuts treated with the microbial inoculants 1, 7, and 25, compared with the control group, the seedlings were more robust and the number of diseased plants was significantly reduced; the seedling disease rate in the control group was more than 5 times that of the treatment groups. The above results indicate that the use of the quality-improving and nitrogen-fixing coupled microbial inoculant has a significant effect of promoting crop resistance to continuous cropping obstacles.
2. Use in soybeans: fields with many years of continuous soybean cultivation were selected to conduct demonstration trials on crop resistance to continuous cropping obstacles. The above microbial inoculants 1, 7, and 25 were respectively mixed with the basal fertilizer at soybean sowing and applied to the field by unmanned aerial vehicles, with a cumulative amount of the microbial inoculants of 80 to 100 billion viable cells per mu of land. Plots without any of the above microbial inoculants were set as controls, and all other management followed conventional field practices. After sowing, irregular and continuous investigations were carried out on the soybeans. The investigation results showed that in the soybeans treated with the microbial inoculants 1, 7, and 25, compared with the control group, the seedlings were more robust and the number of diseased plants was significantly reduced; the seedling disease rate in the control group was more than 5 times that of the treatment groups. The above results indicate that the use of the quality-improving and nitrogen-fixing coupled microbial inoculant has a significant effect of promoting crop resistance to continuous cropping obstacles.
3. Use in other crops such as peas, broad beans, common beans, cowpeas, and potatoes: it also achieved results similar to those obtained for peanuts and soybeans described above in terms of significantly promoting crop resistance to continuous cropping obstacles.

### Example 32: Use of the Quality-Improving and Nitrogen-Fixing Coupled Microbial Inoculant - Promoting Yield Enhancement of Soybeans in Saline-Alkali Land

**Taking the above microbial inoculants 1, 7, and 25 as examples, the use of the quality-improving and nitrogen-fixing coupled microbial inoculant in promoting yield enhancement of soybeans in saline-alkali land is described below.**

Demonstration trials were conducted on saline-alkali land with a pH value ranging from 8.2 to 9.2. The above microbial inoculants 1, 7, and 25 were respectively applied to the field by unmanned aerial vehicles before soybean sowing, with a cumulative amount of the microbial inoculants of 80 to 100 billion viable cells per mu of land. Plots without any of the above microbial inoculants were set as controls, and all other management followed conventional field practices. At harvest, yield measurement and investigation were carried out. The yield measurement and investigation results showed that in the soybeans treated with the microbial inoculants 1, 7, and 25, the yield per unit area was increased by over 10%. The above results indicate that the use of the quality-improving and nitrogen-fixing coupled microbial inoculant has a significant effect of promoting yield enhancement of soybeans in saline-alkali land.

### Example 33: Use of the Quality-Improving and Nitrogen-Fixing Coupled Microbial Inoculant - Preparing a Quality-Improving and Nitrogen-Fixing Coupled Microbial Compound Fertilizer

**Taking the above microbial inoculants 1, 7, and 25 as examples, the use of the quality-improving and nitrogen-fixing coupled microbial inoculant for preparing a quality-improving and nitrogen-fixing coupled microbial compound fertilizer is described below.**

A compound fertilizer was respectively blended with the quality-improving and nitrogen-fixing coupled microbial inoculants 1, 7, and 25 according to a conventional amount of the compound fertilizer and an amount of the quality-improving and nitrogen-fixing coupled microbial inoculant of not less than 100 billion viable cells per mu of land; then, quality-improving and nitrogen-fixing coupled microbial inoculant were adsorbed and immobilized onto the compound fertilizer by a conventional physical method to prepare 3 kinds of quality-improving and nitrogen-fixing coupled microbial compound fertilizers 1, 7, and 25. The above compound fertilizer was an inorganic compound fertilizer, which could be purchased commercially, or could be prepared from inorganic fertilizers such as nitrogen fertilizer, phosphate fertilizer, and potassium fertilizer as raw materials according to common proportions.

The above 3 quality-improving and nitrogen-fixing coupled microbial compound fertilizers 1, 7, and 25 were applied in the production of leguminous crops such as peanuts and soybeans, as seed fertilizer at sowing. Plots treated with the same compound fertilizer were set as controls. Continuous investigation was carried out after sowing. The investigation results showed that the above 3 quality-improving and nitrogen-fixing coupled microbial compound fertilizers 1, 7, and 25 significantly promoted nodulation and nitrogen fixation, with the number of nodules increased by 3.5 times to 13 times, the nitrogenase activity per plant increased by over 10 times, and the yield per unit area increased by over 6.5%, reaching a significant yield enhancement level, exhibiting the main characteristics of the quality-improving and nitrogen-fixing coupled microbial inoculant described in the above examples.

The above results indicate that the quality-improving and nitrogen-fixing coupled microbial inoculant can be used for preparing quality-improving and nitrogen-fixing coupled microbial compound fertilizers, and simultaneously has the effects of both the compound fertilizer and the quality-improving and nitrogen-fixing coupled microbial inoculant.

### Example 34: Use of the Quality-Improving and Nitrogen-Fixing Coupled Microbial Inoculant - Preparing a Quality-Improving and Nitrogen-Fixing Coupled Microbial Organic Fertilizer

**Taking the above microbial inoculants 1, 7, and 25 as examples, the use of the quality-improving and nitrogen-fixing coupled microbial inoculant for preparing a quality-improving and nitrogen-fixing coupled microbial bio-organic fertilizer is described below.**

A bio-organic fertilizer was respectively blended with the quality-improving and nitrogen-fixing coupled microbial inoculants 1, 7, and 25 according to a conventional amount of the bio-organic fertilizer and an amount of the quality-improving and nitrogen-fixing coupled microbial inoculant of not less than 80 billion viable cells per mu of land; then, quality-improving and nitrogen-fixing coupled microbial inoculant were adsorbed and immobilized onto the bio-organic fertilizer by a conventional physical method to prepare 3 kinds of quality-improving and nitrogen-fixing coupled microbial bio-organic fertilizers 1, 7, and 25. The above bio-organic fertilizer could be purchased commercially.

The above 3 quality-improving and nitrogen-fixing coupled microbial compound fertilizers 1, 7, and 25 were applied in the production of leguminous crops such as peanuts and soybeans, as seed fertilizer at sowing. Plots treated with the same bio-organic fertilizer without the microbial inoculants were set as controls. Continuous investigation was carried out after sowing. The investigation results showed that the above 3 quality-improving and nitrogen-fixing coupled microbial bio-organic fertilizers 1, 7, and 25 significantly promoted nodulation and nitrogen fixation, with the number of nodules increased by over 2 times, the nitrogenase activity per plant increased by over 5 times, and the yield per unit area increased by 6%-40%, reaching a significant yield enhancement level, exhibiting the main characteristics of the quality-improving and nitrogen-fixing coupled microbial inoculant described in the above examples.

The above results indicate that the quality-improving and nitrogen-fixing coupled microbial inoculant can be used for preparing quality-improving and nitrogen-fixing coupled microbial bio-organic fertilizers, and simultaneously has the effects of both the bio-organic fertilizer and the quality-improving and nitrogen-fixing coupled microbial inoculant.

### Example 35: Use of the Quality-Improving and Nitrogen-Fixing Coupled Microbial Inoculant - Preparing a Quality-Improving and Nitrogen-Fixing Coupled Microbial Micronutrient Fertilizer

**Taking the above microbial inoculants 1, 7, and 25 as examples, the use of the quality-improving and nitrogen-fixing coupled microbial inoculant for preparing a quality-improving and nitrogen-fixing coupled microbial micronutrient fertilizer is described below.**

A micronutrient fertilizer was respectively blended with the quality-improving and nitrogen-fixing coupled microbial inoculants 1, 7, and 25 according to a conventional amount of the micronutrient fertilizer and an amount of the quality-improving and nitrogen-fixing coupled microbial inoculant of not less than 80 billion viable cells per mu of land, and then they were uniformly mixed by a conventional physical method to prepare 3 kinds of quality-improving and nitrogen-fixing coupled microbial-micronutrient fertilizers 1, 7, and 25. The above micronutrient fertilizer could be purchased commercially.

The above 3 quality-improving and nitrogen-fixing coupled microbial micronutrient fertilizers 1, 7, and 25 were applied in the production of leguminous crops such as peanuts and soybeans, as seed fertilizer at sowing. Plots treated with the same bio-organic fertilizer without the microbial inoculants were set as controls. Continuous investigation was carried out after sowing. The investigation results showed that the above 3 quality-improving and nitrogen-fixing coupled microbial micronutrient fertilizers 1, 7 and 25 significantly promoted nodulation and nitrogen fixation, with the number of nodules increased by over 2 times, the nitrogenase activity per plant increased by over 5 times, and the yield per unit area increased by over 6.5%, reaching a significant yield enhancement level, exhibiting the main characteristics of the quality-improving and nitrogen-fixing coupled microbial inoculant described in the above examples.

The above results indicate that the quality-improving and nitrogen-fixing coupled microbial inoculant can be used for preparing quality-improving and nitrogen-fixing coupled microbial micronutrient fertilizers, and simultaneously has the effects of both the micronutrient fertilizer and the quality-improving and nitrogen-fixing coupled microbial inoculant.

### Example 36: Use of the Quality-Improving and Nitrogen-Fixing Coupled Microbial Inoculant - Preparing a Quality-Improving and Nitrogen-Fixing Coupled Microbial Moisture-Retaining Fertilizer

**Taking the above microbial inoculants 1, 7, and 25 as examples, the use of the quality-improving and nitrogen-fixing coupled microbial inoculant for preparing a quality-improving and nitrogen-fixing coupled microbial moisture-retaining fertilizer is described below.**

A water-retaining agent was respectively blended with the quality-improving and nitrogen-fixing coupled microbial inoculants 1, 7, and 25 according to a conventional amount of the water-retaining agent and an amount of the quality-improving and nitrogen-fixing coupled microbial inoculant of not less than 80 billion viable cells per mu of land, and then they were uniformly mixed by a conventional physical method, or quality-improving and nitrogen-fixing coupled microorganisms were adsorbed and immobilized onto the water-retaining agent by a conventional physical method to prepare 3 kinds of quality-improving and nitrogen-fixing coupled microbial moisture-retaining fertilizers 1, 7, and 25. The above water-retaining agent could be purchased commercially.

The above 3 quality-improving and nitrogen-fixing coupled microbial-water-retaining fertilizers 1, 7, and 25 were applied in the production of leguminous crops such as peanuts and soybeans, as seed fertilizer at sowing. Plots treated with the same water-retaining agent without the microbial inoculants were set as controls. Continuous investigation was carried out after sowing. The investigation results showed that the above 3 quality-improving and nitrogen-fixing coupled microbial moisture-retaining fertilizers 1, 7 and 25 significantly promoted nodulation and nitrogen fixation, with the number of nodules increased by over 2.5 times, the nitrogenase activity per plant increased by over 6 times, and the yield per unit area increased by over 7.5%, reaching a significant yield enhancement level, exhibiting the main characteristics of the quality-improving and nitrogen-fixing coupled microbial inoculant described in the above examples.

The above results indicate that the quality-improving and nitrogen-fixing coupled microbial inoculant can be used for preparing quality-improving and nitrogen-fixing coupled microbial moisture-retaining fertilizers, and simultaneously has the effects of both the water-retaining agent and the quality-improving and nitrogen-fixing coupled microbial inoculant.

### Example 37: Use of the Quality-Improving and Nitrogen-Fixing Coupled Microbial Inoculant - Preparing a Quality-Improving and Nitrogen-Fixing Coupled Microbial Inorganic-Organic Compound Fertilizer

**Taking the above microbial inoculants 1, 7, and 25 as examples, the use of the quality-improving and nitrogen-fixing coupled microbial inoculant for preparing a quality-improving and nitrogen-fixing coupled microbial inorganic-organic compound fertilizer is described below.**

An inorganic-organic compound fertilizer was respectively blended with the quality-improving and nitrogen-fixing coupled microbial inoculants 1, 7, and 25 according to a conventional amount of the inorganic-organic compound fertilizer and an amount of the quality-improving and nitrogen-fixing coupled microbial inoculant of not less than 80 billion viable cells per mu of land; then, quality-improving and nitrogen-fixing coupled microbial inoculant were adsorbed and immobilized onto the inorganic-organic compound fertilizer by a conventional physical method to prepare 3 kinds of quality-improving and nitrogen-fixing coupled microbial inorganic-organic compound fertilizers 1, 7, and 25. The above inorganic-organic compound fertilizer could be purchased commercially, or could be prepared by separately purchasing an inorganic compound fertilizer and a bio-organic fertilizer and blending them according to conventional proportions.

The above 3 quality-improving and nitrogen-fixing coupled microbial inorganic-organic compound fertilizers 1, 7, and 25 were applied in the production of leguminous crops such as peanuts and soybeans, as seed fertilizer at sowing. Plots treated with the same inorganic-organic compound fertilizer were set as controls. Continuous investigation was carried out after sowing. The investigation results showed that the above 3 quality-improving and nitrogen-fixing coupled microbial inorganic-organic compound fertilizers 1, 7, and 25 significantly promoted nodulation and nitrogen fixation, with the number of nodules increased by over 2.3 times, the nitrogenase activity per plant increased by over 5 times, and the yield per unit area increased by over 8%, reaching a significant yield enhancement level, exhibiting the main characteristics of the quality-improving and nitrogen-fixing coupled microbial inoculant described in the above examples.

The above results indicate that the quality-improving and nitrogen-fixing coupled microbial inoculant can be used for preparing quality-improving and nitrogen-fixing coupled microbial inorganic-organic compound fertilizers, and simultaneously has the effects of both the inorganic-organic compound fertilizer and the quality-improving and nitrogen-fixing coupled microbial inoculant.

### Example 38: Use of the Quality-Improving and Nitrogen-Fixing Coupled Microbial Inoculant - Preparing a Quality-Improving and Nitrogen-Fixing Coupled Microbial Rhizobium Fertilizer

**Taking the above microbial inoculants 1, 7, and 25 as examples, the use of the quality-improving and nitrogen-fixing coupled microbial inoculant for preparing a quality-improving and nitrogen-fixing coupled microbial rhizobium fertilizer is described below.**

A rhizobium fertilizer was respectively blended with the quality-improving and nitrogen-fixing coupled microbial inoculants 1, 7, and 25 according to a conventional amount of the rhizobium fertilizer and an amount of the quality-improving and nitrogen-fixing coupled microbial inoculant of not less than 80 billion viable cells per mu of land, and then they were uniformly mixed by a conventional physical method to prepare 3 kinds of quality-improving and nitrogen-fixing coupled microbial-rhizobium fertilizers 1, 7, and 25. The above rhizobium fertilizer, which may also be referred to as a rhizobium inoculant, could be purchased commercially, or could be obtained by isolating from fresh legume nodules and then scaling up through culture.

The above 3 quality-improving and nitrogen-fixing coupled microbial rhizobium fertilizers 1, 7, and 25 were applied in the production of leguminous crops such as peanuts and soybeans, as seed fertilizer at sowing. Plots treated with the same rhizobium fertilizer were set as controls. Continuous investigation was carried out after sowing. The investigation results showed that the above 3 quality-improving and nitrogen-fixing coupled microbial rhizobium fertilizers 1, 7, and 25 significantly promoted nodulation and nitrogen fixation, with the number of nodules increased by over 2 times, the nitrogenase activity per plant increased by over 3 times, and the yield per unit area increased by over 6%, reaching a significant yield enhancement level, exhibiting the main characteristics of the quality-improving and nitrogen-fixing coupled microbial inoculant described in the above examples.

The above results indicate that the quality-improving and nitrogen-fixing coupled microbial inoculant can be used for preparing quality-improving and nitrogen-fixing coupled microbial rhizobium fertilizers, and simultaneously has the effects of both the rhizobium fertilizer and the quality-improving and nitrogen-fixing coupled microbial inoculant.

## Claims

1. A quality-improving and nitrogen-fixing coupled microbial inoculant, wherein the quality-improving and nitrogen-fixing coupled microbial inoculant is a microbial composition that possesses not only a quality-improving and nitrogen-fixing coupled effect to improve quality of leguminous crop products but also effects of regulating and increasing abundance of rhizobia in rhizosphere of leguminous crops and increasing the number of nodules formed on the leguminous crops, and contains all gene sequences or 4 or more thereof among nucleotide sequences set forth in SEQ ID NOs: 1-12.

2. The quality-improving and nitrogen-fixing coupled microbial inoculant according to claim 1, wherein the quality-improving and nitrogen-fixing coupled microbial inoculant is a composition of 3 kinds or more microorganismsand contains 4, 5, 6, 7, 8, 9, 10, 11, 12, or more gene sequences among the nucleotide sequences set forth in SEQ ID NOs: 1-12.

3. The quality-improving and nitrogen-fixing coupled microbial inoculant according to claim 1 or 2, wherein sources of the microorganisms comprise, but are not limited to, bacteria of *Bacillus amyloliquefaciens*, *Bacillus subtilis*, *Bacillus licheniformis*, *Brevibacillus laterosporus*, *Bacillus mucilaginosus*, *Bacillus velezensis*, *Bacillus siamensis*, *Paenibacillus polymyxa*, *Paenibacillus timonensis*, *Pseudomonas fluorescens*, *Pseudomonas mendocina*, *Enterobacter ludwigii*, *Microbacterium proteolyticum*, *Leclercia adecarboxylata*, *Serratia marcescens*, *Empedobacter sp.*, *Priestia megaterium*, and *Stenotrophomonas maltophilia*.

4. The quality-improving and nitrogen-fixing coupled microbial inoculant according to claim 1, wherein the gene sequences set forth in SEQ ID NOs: 1-12 may exhibit a certain degree of variation in different strains; provided that the degree of variation is relatively small, not more than 10% base variations, and corresponding biological activity is retained, these sequence variants constitute functional equivalents of the DNA sequences set forth in SEQ ID NOs: 1-12, and provided that the microbial composition contains all of the gene sequences or 4 or more thereof among the nucleotide sequences set forth in SEQ ID NOs: 1-12, or the functional equivalents of the gene sequences, and possesses not only the quality-improving and nitrogen-fixing coupled effect of the microbial inoculant to improve quality of leguminous crop products but also the effects of regulating and increasing abundance of rhizobia in rhizosphere of leguminous crops and increasing the number of nodules formed on the leguminous crops, the microbial composition constitutes the quality-improving and nitrogen-fixing coupled microbial inoculant of claim 1.

5. The quality-improving and nitrogen-fixing coupled microbial inoculant according to claim 1, wherein the quality-improving and nitrogen-fixing coupled microbial inoculant is capable of improving the quality of leguminous crop products by increasing content of one or more nutritional and functional components, such as protein, glutamic acid, peanut resveratrol, and soybean isoflavone.

6. The quality-improving and nitrogen-fixing coupled microbial inoculant according to claim 1, wherein the quality-improving and nitrogen-fixing coupled microbial inoculant possesses an alleviating effect on one or more soil-borne diseases such as pod rot in peanuts, stay-green syndrome in soybeans, and southern blight, bacterial wilt, root rot, and *Sclerotinia* disease in the leguminous crops;
and/or reduces an aflatoxin level of crop products, thereby improving quality and safety of the leguminous crop products;
and/or reduces spots on peanut pod surfaces.

7. The quality-improving and nitrogen-fixing coupled microbial inoculant according to claim 1, wherein the quality-improving and nitrogen-fixing coupled microbial inoculant is a composition of three or more of the following microbial strains: *Bacillus subtilis* with an accession number of CCTCC NO: M 20231597, *Bacillus velezensis* with an accession number of CCTCC NO: M 20231600, *Brevibacillus laterosporus* with an accession number of CCTCC NO: M 20231808, *Bacillus amyloliquefaciens* with an accession number of CCTCC NO: M 20231810, *Enterobacter ludwigii* with an accession number of CCTCC NO: M 20231812, and *Microbacterium proteolyticum* with an accession number of

8. The quality-improving and nitrogen-fixing coupled microbial inoculant according to claim 1, wherein the quality-improving and nitrogen-fixing coupled microbial inoculant is a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with one or more of the following three strains: *Bacillus subtilis* with an accession number of CCTCC NO: M 20231597, *Bacillus velezensis* with an accession number of CCTCC NO: M 20231600, and *Microbacterium proteolyticum* with an accession number of CCTCC NO: M 20231814.

9. The quality-improving and nitrogen-fixing coupled microbial inoculant according to claim 1, wherein the quality-improving and nitrogen-fixing coupled microbial inoculant is a combination of *Brevibacillus laterosporus* with an accession number of CCTCC NO: M 20231808, *Bacillus amyloliquefaciens* with an accession number of CCTCC NO: M 20231810, and *Enterobacter ludwigii* with an accession number of CCTCC NO: M 20231812, with other microorganisms, such that a combined microbial inoculant contains all of the gene sequences or 4 or more thereof among the nucleotide sequences set forth in SEQ ID NOs: 1-12, or functional equivalents thereof, and possesses not only the quality-improving and nitrogen-fixing coupled effect of the microbial inoculant but also the effects of regulating and increasing abundance of rhizobia in rhizosphere of leguminous crops and increasing the number of nodules formed on the leguminous crops, constituting the quality-improving and nitrogen-fixing coupled microbial inoculant of claim 1.

10. The quality-improving and nitrogen-fixing coupled microbial inoculant according to claim 1, wherein a percentage of viable cells of any single strain in the quality-improving and nitrogen-fixing coupled microbial inoculant is greater than or equal to 1%.

11. Use of the quality-improving and nitrogen-fixing coupled microbial inoculant of claim 1 in production of leguminous crops for any one of the following purposes: improving quality and safety of leguminous crop products; promoting nodulation and nitrogen fixation of the leguminous crops; increasing a yield per unit area of the leguminous crops; recruiting indigenous rhizobia and increasing abundance of rhizobia in rhizosphere soil of the leguminous crops; promoting earlier nodulation and prolonging a nodulation and nitrogen fixation period of the leguminous crops; preventing nutrient deficiency and premature senescence of the leguminous crops at maturity; increasing the number of pods of the leguminous crops; increasing fullness of pods and reducing a rate of shriveled pods of the leguminous crops; promoting earlier flowering and earlier podding of the leguminous crops; reducing occurrence of pod rot in peanuts; reducing occurrences of bacterial wilt in the leguminous crops; reducing occurrences of powdery mildew in the leguminous crops; reducing occurrences of leaf spot in the leguminous crops; reducing occurrences of root-knot nematode disease in the leguminous crops; reducing occurrences of root rot in the leguminous crops; reducing occurrences of *Sclerotinia* disease in the leguminous crops; reducing occurrences of downy mildew in the leguminous crops; reducing occurrences of *Fusarium* wilt in the leguminous crops, and reducing occurrences of southern blight in the leguminous crops; reducing severity of stay-green syndrome in soybeans; promoting carbon emission reduction in the leguminous crops, and facilitating soil improvement; promoting an increase in total biomass of the leguminous crops; reducing spots on peanut pod surfaces and improving marketability; and promoting yield enhancement of soybeans in saline-alkali land.

12. Use of the quality-improving and nitrogen-fixing coupled microbial inoculant of claim 1 for preparing a quality-improving and nitrogen-fixing coupled microbial compound fertilizer, or a quality-improving and nitrogen-fixing coupled microbial organic fertilizer, or a quality-improving and nitrogen-fixing coupled microbial micronutrient fertilizer, or a quality-improving and nitrogen-fixing coupled microbial moisture-retaining fertilizer, or a quality-improving and nitrogen-fixing coupled microbial inorganic-organic compound fertilizer, or a quality-improving and nitrogen-fixing coupled microbial rhizobium fertilizer, or a quality-improving and nitrogen-fixing coupled microbial seed coating agent/seed dressing agent/seed soaking agent to simultaneously exert effects of the quality-improving and nitrogen-fixing coupled microbial inoculant in production.

13. A quality-improving and nitrogen-fixing coupled microbial compound fertilizer, or a quality-improving and nitrogen-fixing coupled microbial organic fertilizer, or a quality-improving and nitrogen-fixing coupled microbial micronutrient fertilizer, or a quality-improving and nitrogen-fixing coupled microbial moisture-retaining fertilizer, or a quality-improving and nitrogen-fixing coupled microbial inorganic-organic compound fertilizer, or a quality-improving and nitrogen-fixing coupled microbial rhizobium fertilizer, or a quality-improving and nitrogen-fixing coupled microbial seed coating agent/seed dressing agent/seed soaking agent prepared by using the quality-improving and nitrogen-fixing coupled microbial inoculant of claim 1.

14. A method for preparing a quality-improving and nitrogen-fixing coupled microbial compound fertilizer, or a quality-improving and nitrogen-fixing coupled microbial organic fertilizer, or a quality-improving and nitrogen-fixing coupled microbial micronutrient fertilizer, or a quality-improving and nitrogen-fixing coupled microbial moisture-retaining fertilizer, or a quality-improving and nitrogen-fixing coupled microbial inorganic-organic compound fertilizer, or a quality-improving and nitrogen-fixing coupled microbial rhizobium fertilizer by using the quality-improving and nitrogen-fixing coupled microbial inoculant of claim 1, wherein
the method for preparing the quality-improving and nitrogen-fixing coupled microbial compound fertilizer comprises: blending a compound fertilizer with the quality-improving and nitrogen-fixing coupled microbial inoculant according to a conventional amount of the compound fertilizer and an amount of the quality-improving and nitrogen-fixing coupled microbial inoculant of not less than 80 billion viable cells per mu of land, and then adsorbing and immobilizing quality-improving and nitrogen-fixing coupled microbial inoculant onto compound fertilizer particles by a physical method to prepare the quality-improving and nitrogen-fixing coupled microbial compound fertilizer;
the method for preparing the quality-improving and nitrogen-fixing coupled microbial organic fertilizer comprises: blending an organic fertilizer with the quality-improving and nitrogen-fixing coupled microbial inoculant according to a conventional amount of the organic fertilizer and an amount of the quality-improving and nitrogen-fixing coupled microbial inoculant of not less than 80 billion viable cells per mu of land, and then adsorbing and immobilizing quality-improving and nitrogen-fixing coupled microbial inoculant onto organic fertilizer particles by a physical method to prepare the quality-improving and nitrogen-fixing coupled microbial organic fertilizer;
the method for preparing the quality-improving and nitrogen-fixing coupled microbial micronutrient fertilizer comprises: blending a micronutrient fertilizer with the quality-improving and nitrogen-fixing coupled microbial inoculant according to a conventional amount of the micronutrient fertilizer and an amount of the quality-improving and nitrogen-fixing coupled microbial inoculant of not less than 80 billion viable cells per mu of land, uniformly mixing them by a physical method, and adsorbing and immobilizing quality-improving and nitrogen-fixing coupled microbial inoculant onto micronutrient fertilizer particles to prepare the quality-improving and nitrogen-fixing coupled microbial micronutrient fertilizer;
the method for preparing the quality-improving and nitrogen-fixing coupled microbial moisture-retaining fertilizer comprises: blending a water retaining agent with the quality-improving and nitrogen-fixing coupled microbial inoculant according to a conventional amount of the water retaining agent and an amount of the quality-improving and nitrogen-fixing coupled microbial inoculant of not less than 80 billion viable cells per mu of land, and then uniformly mixing them by a conventional physical method, or adsorbing and immobilizing quality-improving and nitrogen-fixing coupled microorganisms onto the water retaining agent to prepare the quality-improving and nitrogen-fixing coupled microbial moisture-retaining fertilizer;
the method for preparing the quality-improving and nitrogen-fixing coupled microbial rhizobium fertilizer comprises: blending a rhizobium fertilizer with the quality-improving and nitrogen-fixing coupled microbial inoculant according to a conventional amount of the rhizobium fertilizer and an amount of the quality-improving and nitrogen-fixing coupled microbial inoculant of not less than 80 billion viable cells per mu of land, then uniformly mixing them by a conventional physical method, or uniformly mixing them and then immobilizing them onto carrier particles to prepare the quality-improving and nitrogen-fixing coupled microbial rhizobium fertilizer;
the method for preparing the quality-improving and nitrogen-fixing coupled microbial inorganic-organic compound fertilizer comprises: blending an inorganic-organic compound fertilizer with the quality-improving and nitrogen-fixing coupled microbial inoculant according to a conventional amount of the inorganic-organic compound fertilizer and an amount of the quality-improving and nitrogen-fixing coupled microbial inoculant of not less than 80 billion viable cells per mu of land, and then adsorbing and immobilizing quality-improving and nitrogen-fixing coupled microbial inoculant onto inorganic-organic particles by a physical method to prepare the quality-improving and nitrogen-fixing coupled microbial inorganic-organic compound fertilizer; and
the method for preparing the quality-improving and nitrogen-fixing coupled microbial seed coating agent/seed dressing agent/seed soaking agent comprises: blending a seed coating agent or seed dressing agent or seed soaking agent with the quality-improving and nitrogen-fixing coupled microbial inoculant according to an amount of the seed coating agent or seed dressing agent or seed soaking agent and an amount of the quality-improving and nitrogen-fixing coupled microbial inoculant of not less than 80 billion viable cells per mu of land, and then uniformly mixing them by a conventional physical method to prepare the quality-improving and nitrogen-fixing coupled microbial seed coating agent/seed dressing agent/seed soaking agent.

15. Use of the quality-improving and nitrogen-fixing coupled microbial compound fertilizer, or the quality-improving and nitrogen-fixing coupled microbial organic fertilizer, or the quality-improving and nitrogen-fixing coupled microbial micronutrient fertilizer, or the quality-improving and nitrogen-fixing coupled microbial moisture-retaining fertilizer, or the quality-improving and nitrogen-fixing coupled microbial inorganic-organic compound fertilizer, or the quality-improving and nitrogen-fixing coupled microbial rhizobium fertilizer prepared according to claim 14 in crop production for any one of the following purposes: improving quality and safety of leguminous crop products; promoting nodulation and nitrogen fixation of leguminous crops; increasing a yield per unit area of the leguminous crops; recruiting indigenous rhizobia and increasing abundance of rhizobia in rhizosphere soil of the leguminous crops; promoting earlier nodulation and prolonging a nodulation and nitrogen fixation period of the leguminous crops; preventing nutrient deficiency and premature senescence of the leguminous crops at maturity; increasing the number of pods of the leguminous crops; increasing fullness of pods and reducing a rate of shriveled pods of the leguminous crops; promoting earlier flowering and earlier podding of the leguminous crops; reducing occurrence of pod rot in peanuts; reducing occurrences of bacterial wilt in the leguminous crops; reducing occurrences of powdery mildew in the leguminous crops; reducing occurrences of leaf spot in the leguminous crops; reducing occurrences of root-knot nematode disease in the leguminous crops; reducing occurrences of root rot in the leguminous crops; reducing occurrences of *Sclerotinia* disease in the leguminous crops; reducing occurrences of downy mildew in the leguminous crops; reducing occurrences of ***Fusarium*** wilt in the leguminous crops, and reducing occurrences of southern blight in the leguminous crops; reducing severity of stay-green syndrome in soybeans; promoting carbon emission reduction in the leguminous crops, and facilitating soil improvement; promoting an increase in total biomass of the leguminous crops; reducing spots on peanut pod surfaces and improving marketability; and promoting yield enhancement of soybeans in saline-alkali land.

16. A method for production of leguminous crops, comprising: selecting and preparing a microbial inoculant, such that the microbial inoculant, upon analytical determination, contains all gene sequences or 4 or more thereof among nucleotide sequences set forth in SEQ ID NOs: 1-12, and possesses not only a quality-improving and nitrogen-fixing coupled effect but also effects of regulating and increasing abundance of rhizobia in rhizosphere of the leguminous crops and increasing the number of nodules formed on the leguminous crops; and applying the microbial inoculant to crops for quality improvement, nitrogen fixation, and green yield enhancement.

17. The method according to claim 16, wherein the gene sequences set forth in SEQ ID NOs: 1-12 may exhibit not more than 10% base variations in different strains; provided that the sequences share more than 90% identity with corresponding DNA sequences and retain corresponding biological activity, these sequence variants constitute functional equivalents of the DNA sequences set forth in SEQ ID NOs: 1-12; and provided that the microbial inoculant contains all of the gene sequences or 4 or more thereof among the nucleotide sequences set forth in SEQ ID NOs: 1-12, or the functional equivalents of these gene sequences, and possesses not only the quality-improving and nitrogen-fixing coupled effect of the microbial inoculant but also the effects of regulating and increasing abundance of rhizobia in rhizosphere of leguminous crops and increasing the number of nodules formed on the leguminous crops, the microbial inoculant constitutes the quality-improving and nitrogen-fixing coupled microbial inoculant of claim 1, and is applied in crops for quality improvement, nitrogen fixation, and green yield enhancement.
